# EUROPÄISCHE PATENTSCHRIFT

(11) **EP 2 099 781 B1**
(45) Veröffentlichungstag und Bekanntmachung des Hinweises auf die Patenterteilung: **24.10.2012**
(21) Anmeldenummer: 07846652.1
(22) Anmeldetag: 17.11.2007
(51) Int. Cl.: C07D 401/12, C07D 401/14, C07D 413/12, C07D 417/12, A61K 31/4439, A61P 9/00

(54) **SUBSTITUIERTE 4-AMINO-3,5-DICYANO-2-THIOPYRIDINE UND IHRE VERWENDUNG**
SUBSTITUTED 4-AMINO-3,5-DICYANO-2-THIOPYRIDINES AND USE THEREOF
4-AMINO-3,5-DICYANO-2-THIOPYRIDINE SUBSTITUÉE ET SON UTILISATION

(30) Priorität: 01.12.2006 DE 102006056739
(43) Veröffentlichungstag der Anmeldung: 16.09.2009
(73) Patentinhaber: Bayer Intellectual Property GmbH, 40789 Monheim (DE)
(72) Erfinder: NELL, Peter, 42115 Wuppertal (DE); DIEDRICHS, Nicole, 42553 Velbert (DE); ALBRECHT-KÜPPER, Barbara, 42289 Wülfrath (DE); VAKALOPOULOS, Alexandros, 40721 Hilden (DE); SÜSSMEIER, Frank, 42277 Wuppertal (DE); KELDENICH, Jörg, 42113 Wuppertal (DE)
(74) Vertreter: Bayer Intellectual Property GmbH
(86) Internationale Anmeldenummer: PCT/EP2007/009963
(87) Internationale Veröffentlichungsnummer: WO 2008/064789

(56) Entgegenhaltungen:
- WO-A-01/62233
- WO-A-02/070485
- BEUKERS MARGOT W ET AL: "New, non-adenosine, high-potency agonists for the human adenosine A2B receptor with an improved selectivity profile compared to the reference agonist n-ethylcarboxamidoadenosine" JOURNAL OF MEDICINAL CHEMISTRY, AMERICAN CHEMICAL SOCIETY. WASHINGTON, US, Bd. 47, Nr. 15, 15. Juli 2004 (2004-07-15), Seiten 3707-3709, XP002390870 ISSN: 0022-2623

## Beschreibung

Die vorliegende Anmeldung betrifft neue, substituierte 4-Amino-3,5-dicyano-2-thiopyridin-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, vorzugsweise zur Behandlung und/oder Prävention von Hypertonie und anderen kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intracellulären cAMP, während die Bindung des Adenosin an die A1- oder A3-Rezeptoren über eine Hemmung der Adenylatzyklase eine Abnahme des intrazellulären cAMP-Gehahs bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A2b-Rezeptoren durch Adenosin oder spezifische A2b-Agonisten führt über die Erweiterung von Gefäßen zu einer Blutdrucksertkung. Die Blutdrucksenkung ist von einem refleklorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultien somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

In Adipozyten bewirkt die Aktivierung von A1- und A2b-Rezeptoren eine Inhibition der Lipolyse. Die kombinierte Wirkung von A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles. "Cloning, expression, and characterization of the unique bovine A1 adenosine receptor. Studies on the ligand binding site by site-directed mutagenesis", J. Biol. Chem. 267 (1992), Seiten 10764-10770. deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9, deren Offenbarung hiermit im vollen Umfang durch Bezugnahme eingeschlossen ist).

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet. In klinischer Entwicklung befindliche Verbindungen dieser Art eignen sich bislang nur zur intravenösen Applikation.

In WO 01/25210 und WO 02/070485 werden substituierte 2-Thio-3,5-dicyano-4-aryl-6-aminopyridine als Adenosinrezeptor-Liganden für die Behandlung von Erkrankungen beschrieben. In WO 03/053441 werden spezifisch substituierte 2-Thio-3,5-dicyano-4-phenyl-6-aminopyridine als selektive Liganden des Adenosin A1-Rezeptors offenbart, und in WO 2006/027142 werden substituierte Phenylaminothiazol-Derivate als duale Adenosin A1/A2b-Agonisten für die Behandlung der Hypertonie und anderer kardiovaskulärer Erkrankungen beansprucht. Allerdings zeigte es sich, dass diese Verbindungen zum Teil hinsichtlich ihrer physikochemischen und/oder pharmakokinetischen Eigenschaften, wie beispielsweise ihrer Löslichkeit in Wasser und anderen physiologischen Medien oder ihres Absorptionsverhaltens im Körper, Nachteile aufweisen.

In WO 01/62233 werden verschiedene Pyridin- und Pyrimidin-Derivate sowie ihre Verwendung als Adenosinrezepior-Modulatoren offenbart. Substituierte 3,5-Dicyanopyridine als Calciumabhängige Kaliumkanalöffner zur Behandlung urologischer Erkrankungen werden in EP 1 302 463-A1 beansprucht. Verschiedene heterocyclisch substituierte Pyridin-Derivate und ihre Verwendung zur Behandlung von Krankheiten werden in WO 99/32117, WO 2004/054505, WO 2005/046603 und WO 2006/034446 beschrieben. In WO 02/50071 werden Aminothiazol-Derivate als Tyrosin-Kinase-Inhibitoren für die Behandlung von Krebs sowie immunologischer und allergischer Erkrankungen offenbart.

Aufgabe der vorliegenden Erfindung ist die Bereitstellung neuer Verbindungen, die als selektive Agonisten des Adenosin A1-Rezeptors oder als selektive duale Agonisten des Adenosin A1- und A2b-Rezeptors wirken, als solche zur Behandlung und/oder Prävention insbesondere von Hypertonie und anderen kardiovaskulären Erkrankungen, des Metabolischen Syndroms, Diabetes und Dyslipidämien sowie zur Organprotektion bei Transplantationen und operativen Eingiffen geeignet sind und darüber hinaus ein verbessertes physikochemisches und/oder pharmakokinetisches Profil gegenüber den aus dem Stand der Technik bekannten Substanzen aufweisen.

Gegenstand der vorliegenden Erfindung sind Verbindungen der Formel (I) 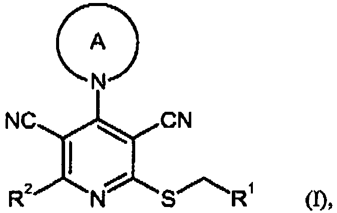 in welcher
- der Ring A: für einen gesättigten 4- bis 7-gliedrigen, N-verknüpften Heterocyclus steht, der ein weiteres Ring-Heteroatom aus der Reihe N, O und S enthalten kann und der
(i) bis zu fünffach, gleich oder verschieden, mit (C₁-C₄)-Alkyl, das seinerseits ein- oder zweifach, gleich oder verschieden, mit Oxo, Hydroxy, (C₁-C₄)-Alkoxy, Amino, Mono-(C₁-C₄)-alkylamino, Di-(C₁-C₄)-alkylamino und/oder (C₃-C₆)-Cycloalkyl substituiert sein kann,
   und/oder
(ii) ein- oder zweifach, gleich oder verschieden, mit Oxo, Thioxo, Hydroxy, (C₁-C₄)-Alkoxy, Amino, Mono-(C₁-C₄)-alkylamino, Di-(C₁-C₄)-alkylamino und/oder (C₃-C₆)-Cycloalkyl
   substituiert sein kann,
- R¹: für (C₆-C₁₀)-Aryl oder 5- bis 10-gliedriges Heteroaryl mit bis zu drei Ring-Heteroatomen aus der Reihe N, O und/oder S steht, welche jeweils
(*i*) ein- oder zweifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Mono-(C₂-C₆)-alkenyl. amino, Carboxyl, (C₁-C₆)-Alkoxycarbonyl, Carbamoyl, Mono-(C₁-C₆)-alkylamino-carbonyl und Di-(C₁-C₆)-alkylaminocarbonyl
   und/oder
(*ii*) mit Pyrrolidino, Piperidino, Morpholino, Piperazino, *N'*-(C₁-C₄)-Alkylpiperazino oder einer Gruppe der Formel -L-R³, worin
   L eine Bindung, NH oder O bedeutet und
   R³ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, (C₁-C₆)-Alkoxycarbonyl und Carboxyl substituiert sein können, substituiert sein können,
und
- R²: für Wasserstoff oder für (C₁-C₆)-Alkyl oder (C₁-C₆)-Alkoxy, welche jeweils mit Hydroxy, (C₁-C₄)-Alkoxy. Carboxyl, (C₁-C₄)-Alkoxycarbonyl oder bis zu dreifach mit Fluor substituien sein können, steht
oder
- R²: für eine Gruppe der Formel -NR⁴R⁵ steht, worin
R⁴ und R⁵ gleich oder verschieden sind und unabhängig voneinander für Wasserstoff oder (C₁-C₆)-Alkyl, das ein- oder zweifach, gleich oder verschieden, mit Hydroxy, (C₁-C₄)-Alkoxy, Amino, Mono-(C₁-C₄)-alkylamino, Di-(C₁-C₄)-alkylamino, Carboxyl, (C₁-C₄)-Alkoxvcarbonyl und/oder einem 4- bis 7-gliedrigen Heterocyclus substituiert sein kann, stehen,
wobei der genannte Heterocyclus ein oder zwei Ring-Heteroatome aus der Reihe N, O und/oder S enthält und seinerseits ein- oder zweifach, gleich oder verschieden, mit (C₁-C₄)-Alkyl, Hydroxy, Oxo und/oder (C₁-C₄)-Alkoxy substituiert sein kann, oder
R⁴ und R⁵ zusammen mit dem Stickstoffatom, an das sie gebunden sind, einen 4- bis 7-gliedrigen Heterocyclus bilden, der ein weiteres Ring-Heteroatom aus der Reihe N, O und S enthalten und ein- oder zweifach, gleich oder verschieden, mit (C₁-C₄)-Alkyl, Hydroxy, Oxo, (C₁-C₄)-Alkoxy, Azetidino, Pyrrolidino, Piperidino und/oder Morpholino substituiert sein kann,
sowie ihre *N*-Oxide, Salze, Solvate, Salze der N-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgeinäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl, (C₁-C₄)-Alkyl und (C₁-C₃)-Alkyl stehen im Rahmen der Erfindung für einen geradkettigen oder verzweigten Alkylrest mit 1 bis 6, 1 bis 4 bzw. 1 bis 3 Kohlenstoffatomen. Bevorzugt ist ein geradkettiger oder verzweigter Alkylrest mit 1 bis 4, besonders bevorzugt mit 1 bis 3 Kohlenstoffatomen. Beispielhaft und vorzugsweise seien genannt: M ethyl, Ethyl, n-Propyl, Isopropyl, n-Butyl, iso-Butyl, sec.-Butyl, tert.-Butyl, 1-Ethylpropyl, n-Pentyl und n-Hexyl.
(C₂-C₆)-Alkenyl steht im Rahmen der Erfindung für einen geradkettigen oder verzweigten Alkenylrest mit 2 bis 6 Kohlenstoffatomen und einer oder zwei Doppelbindungen. Bevorzugt ist ein geradkettiger oder verzweigter Alkenylrest mit 2 bis 4 Kohlenstoffatomen und einer Doppelbindung. Beispielhaft und vorzugsweise seien genannt: Vinyl, Allyl, Isopropenyl, 2-Methylprop-2-en-1-yl, n-But-2-en-1-yl und n-But-3-en-1-yl.
(C₃-C₆)-Cycloalkyl und (C₃-C₅)cycloalkyl stehen im Rahmen der Erfindung für einen monocyclischen, gesättigten Carbocyclus mit 3 bis 6 bzw. 3 bis 5 Ring-Kohlenstoffatomen. Beispielhaft und vorzugsweise seien genannt: Cyclopropyl, Cyclobutyl, Cyclopentyl und Cyclohexyl.
(C₁-C₆)-Alkoxy, (C₁-C₄)-Alkoxy und (C₁-C₄)-Alkoxy stehen im Rahmen der Erfindung für einen geradkettigen oder verzweigten Alkoxyrest mit 1 bis 6, 1 bis 4 bzw. 1 bis 3 Kohlenstoffatomen. Bevorzugt ist ein geradkettiger oder verzweigter Alkoxyrest mit 1 bis 4, besonders bevorzugt mit 1 bis 3 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-(C₁-C₆)-alkylamino, Mono-(C₁-C₄)-alkylamino und Mono-(C₁-C₃)-alkylamino stehen im Rahmen der Erfindung für eine Amino-Gruppe mit einem geradkettigen oder verzweigten Alkylsubstituenten, der 1 bis 6, 1 bis 4 bzw. 1 bis 3 Kohlenstoffatome aufweist. Bevorzugt ist ein geradkettiger oder verzweigter Monoalkylarnino-Rest mit 1 bis 4, besonders bevorzugt mit 1 bis 3 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.
Mono-(C₂-C₆)-alkenylamino steht im Rahmen der Erfindung für eine Amino-Gruppe mit einem geradkettigen oder verzweigten Alkenylsubstituenten, der 2 bis 6 Kohlenstoffatome aufweist. Bevorzugt ist ein geradkettiger oder verzweigter Monoalkenylamino-Rest mit 1 bis 4 Kohlenstoffatomen. Beispielhaft und vorzugsweise seien genannt: Allylamino, 1-Methylprop-2-en-1-ylamino, 2-Methylprop-2-en-1-ylamino, But-2-en-1-ylamino und But-3-en-1-ylamino.
Di-(C₁-C₆)-alkylamino, Di-(C₁-C₄)-alkylamino und Di-(C₁-C₃)-alkylamino stehen im Rahmen der Erfindung für eine Amino-Gruppe mit zwei gleichen oder verschiedenen geradkettigen oder verzweigten Alkylsubstituenten, die jeweils 1 bis 6, 1 bis 4 bzw. 1 bis 3 Kohlenstoffatome aufweisen. Bevorzugt sind geradkettige oder verzweigte Dialkylamino-Reste mit jeweils 1 bis 4, besonders bevorzugt mit jeweils 1 bis 3 Kohlenstoffatomen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N-*Isopropyl-*N*-n-propylamino, *N,N*-Diisopropylamino, *N*-n-Butyl-*N*-methylamino, *N*-tert.-Butyl-*N-*methylamino, *N*-Ethyl-*N*-n-pentylamino und *N*-n-Hexyl-*N*-methylamino.
Mono- bzw. Di-(C₁-C₆)-alkylaminocarbonyl steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 6 Kohlenstoffatomen aufweist. Bevorzugt ist ein Mono- bzw. Dialkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, *n*-Propylaminocarbonyl, Isopropylaminocarbonyl, *n-*Butylaminocarbonyl, *tert*.-Butylaminocarbonyl, *N,N*-Dimethylaminocarbonyl, *N,N*-Diethylaminocarbonyl, *N*-Ethyl-*N*-methylaminocarbonyl, *N*-Methyl-*N*-*n*-propylarninocarbonyl, *N*-*n*-Butyl-*N-*methylaminocarbonyl und *N-tert*.-Butyl-*N*-methylaminocarbonyl.
(C₆-C₁₀)-Aryl steht im Rahmen der Erfindung für einen aromatischen Carbocyclus mit 6 oder 10 Ring-Kohlenstoffatomen. Bevorzugte Arylreste sind Phenyl und Naphthyl.
Ein 4- bis 7-gliedriger Heterocyclus steht im Rahmen der Erfindung für einen gesättigten Heterocyclus mit insgesamt 4 bis 7 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Bevorzugt ist ein 5- oder 6-gliedriger Heterocyclus mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Hexahydroazepinyl und Hexahydro-1,4-diazepinyl. Bevorzugt sind Pyrrolidinyl, Tetrahydrofuranyl, Piperidinyl, Piperazinyl, Tetrahydropyranyl und Morpholinyl.
5- bis 10-gliedriges Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu drei Ring-Heteroatomen aus der Reihe N, O und/oder S wie beispielsweise Furyl, Thienyl, Thiazolyl, Oxazolyl, Isothiazolyl, Isoxazoly!, Pyrazolyl, Imidazolyl, Triazolyl, Oxadiazolyl, Thiadiazolyl, Pyridyl, Pyrimidinyl, Pyridazinyl, Pyrazinyl, Triazinyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Von besonderer Bedeutung im Rahmen der vorliegenden Erfindung sind Verbindungen der Formel (I), in welcher
- R¹: für Phenyl oder 5- oder 6-gliedriges Heteroaryl mit bis zu drei Ring-Heteroatomen aus der Reihe N, O und/oder S steht, welche jeweils
(i) ein- oder zweifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Mono-(C₂-C₆)-alkenyl-amino, Carboxyl, (C₁-C₆)-Alkoxycarbonyl, Carbamoyl, Mono-(C₁-C₆)-alkylamino-carbonyl und Di-(C₁-C₆)-alkylaminocarbonyl
   und/oder
(ii) mit Pyrrolidino, Piperidino, Morpholino, Piperazino, *N*'-(C₁-C₄)-Alkylpiperazino oder einer Gruppe der Formel -L-R³, worin
   L eine Bindung, NH, oder O bedeutet und
   R³ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit einem Rest ausgewählt aus der Reihe Halogen, Nitro, Cyano, (C₁-C₆)-Alkyl. Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, (C₁-C₆)-Alkoxyarbonyl und Carboxyl substituiert sein können, substituiert sind,
oder
- R¹: für *N*-Oxidopyridyl steht,
sowie ihre Salze, Solvate und Solvate der Salze.

Bevorzugt im Rahmen der vorliegenden Erfindung sind Verbindungen der Formel (I), in welcher
- der Ring A: für einen gesättigten. 5- bis 7-gliedrigen, N-verknüpften Heterocyclus steht, der ein weiteres Ring-Heteroatom aus der Reihe N und O enthalten kann und der
(*i*) bis zu fünffach, gleich oder verschieden, mit (C₁-C₃)-Alkyl, das seinerseits ein- oder zweifach, gleich oder verschieden, mit Oxo, Hydroxy, (C₁-C₃)-Alkoxy, Amino, Mono-(C₁-C₃)-alkylamino, Di-(C₁-C₃)-alkylamino und/oder (C₃-C₅)-Cycloalkyl substituiert sein kann,
   und/oder
(*ii*) ein- oder zweifach, gleich oder verschieden, mit Oxo, Hydroxy, (C₁-C₃)-Alkoxy, Amino, Mono-(C₁-C₃)-alkylamino, Di-(C₁-C₃)-alkylamino und/oder (C₃-C₅)-Cycloalkyl
   substituiert sein kann,
- R¹: für Phenyl oder 5- oder 6-gliedriges Heteroaryl mit bis zu drei Ring-Heteroatomen aus der Reihe N, O und/oder S steht, welche jeweils
(*i*) ein- oder zweifach, gleich oder verschieden, mit Fluor, Chlor, Cyano, (C₁-C₄)-Alkyl, Amino, Mono-(C₁-C₄)-alkylamino und/oder Di-(C₁-C₄)-alkylamino
   und/oder
(*ii*) mit Morpholino, *N'*-(C₁-C₄)-Alkylpiperazino oder einer Gruppe der Formel -L-R³, worin
   L eine Bindung oder NH bedeutet und
   R³ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit Fluor, Chlor, Cyano, (C₁-C₄)-Alkyl, Trifluormethyl, (C₁-C₄)-Alkoxy, Trifluormethoxy und/oder Carboxyl substituiert sein können,
substituiert sind,
oder
- R¹: für *N*-Oxidopyridyl steht,
und
- R²: für Wasserstoff oder für (C₁-C₄)-Alkoxy, das bis zu dreifach mit Fluor substituiert sein kann, steht
oder
- R²: für eine Gruppe der Formel -NR⁴R⁵ steht, worin
R⁴ für Wasserstoff oder (C₁-C₄)-Alkyl, das mit Hydroxy, (C₁-C₄)-Alkoxy, Amino, Mono-(C₁-C₄)-alkylamino, Di-(C₁-C₄)-alkylanvno, Carboxyl, (C₁-C₄)-Alkoxycarbonyl oder einem 5- oder 6-gliedrigen Heterocyclus substituiert sein kann, steht,
wobei der genannte Heterocyclus ein oder zwei Ring-Heteroatome aus der Reihe N und/oder O enthält und seinerseits ein- oder zweifach, gleich oder verschieden, mit Methyl, Ethyl, Hydroxy, Methoxy und/oder Ethoxy substituiert sein kann,
R⁵ für Wasserstoff oder Methyl steht oder
R⁴ und R⁵ zusammen mit dem Stickstoffatom, an das sie gebunden sind, einen 5- oder 6-gliedrigen Heterocyclus bilden, der ein weiteres Ring-Heteroatom aus der Reihe N und O enthalten und ein- oder zweifach, gleich oder verschieden, mit Methyl, Ethyl, Hydroxy, Methoxy und/oder Ethoxy substituiert sein kann,
sowie ihre Salze, Solvate und Solvate der Salze.

Besonders bevorzugt im Rahmen der vorliegenden Erfindung sind Verbindungen der Formel (I), in welcher
der Ring A für eine Gruppe der Formel 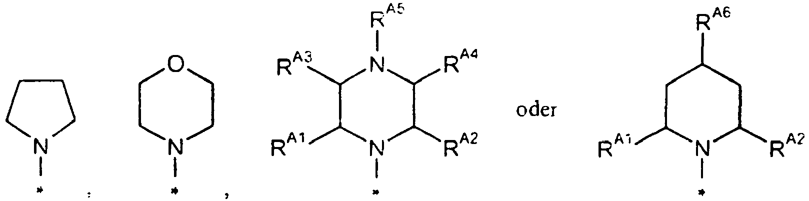 steht, worin
* die Verknüpfungssielle mit dem Pyridin-Ring,
   - R^{A1}, R^{A2}, R^{A3} und R^{A4}: unabhängig voneinander Wasserstoff oder Methyl,
   - R^{A5}: Wasserstoff, Methyl, Ethyl, 2-Hydroxyethyl oder Cyclopropylmethyl
   und
   - R^{A6}: Wasserstoff, Methyl, Ethyl, Cyclopropylmethyl, Hydroxymethyl, 2-Hydroxyethyl, Hydroxy, Methoxy oder Ethoxy
   bedeuten,

- R¹: für Phenyl, Oxazolyl, Thiazolyl oder Pyridyl steht, welche jeweils
(*i*) ein- oder zweifach, gleich oder verschieden, mit Fluor, Chlor, Methyl und/oder Amino
   oder
(*ii*) mit einer Gruppe der Formel -L-R³, worin
   L eine Bindung oder NH bedeutet und
   R³ Phenyl oder Pyridyl bedeutet, welche jeweils ein- oder zweifach, gleich oder verschieden, mit Fluor, Chlor, Cyano, Methyl und/oder Methoxy substituiert sein können,
substituiert sind,
oder
- R¹: für *N*-Oxidopyridyl steht,
und
- R²: für Wasserstoff, Methoxy oder eine Gruppe der Formel -NR⁴R⁵ steht, worin
- R⁴: für Wasserstoff oder (C₁-C₄)-Alkyl, das mit Hydroxy, Amino, Methylamino, Ethylamino, Dimethylamino oder Diethylamino substituiert sein kann, steht,
- R⁵: für Wasserstoff steht
oder
- R⁴ und R⁵: zusammen mit dem Stickstoffatom, an das sie gebunden sind, eine Gruppe der Formel 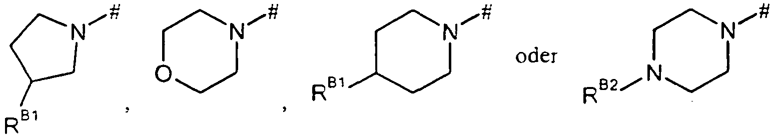 bilden, worin
# die Verknüpfungsstelle mit dem Pyridin-Ring,
R^{B1} Wasserstoff oder Hydroxy und
R^{B2} Wasserstoff oder Methyl bedeuten,
sowie ihre Salze, Solvate und Solvate der Salze.

Weiterer Gegenstand der vorliegenden Erfindung ist ein Verfahren zur Herstellung der erfindungsgemäßen Verbindungen der Formel (I), in welcher R² für NH₂ steht, dadurch gekennzeichnet, dass man die Verbindung der Formel (II) 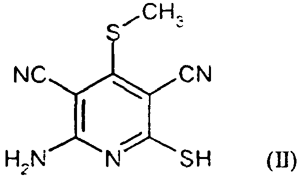 zunächst in einem inerten Lösungsmittel in Gegenwart einer Base mit einer Verbindung der Formel (III) 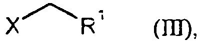 in welcher R¹ die oben angegebene Bedeutung hat und
- X: für eine geeignete Abgangsgruppe, vorzugsweise für Halogen, insbesondere Chlor, Brom oder Iod, oder für Mesylat, Tosylat oder Triflat steht,
zu einer Verbindung der Formel (IV) 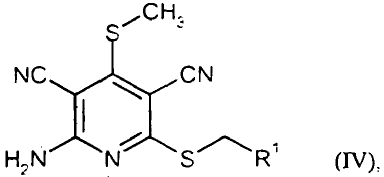 in welcher R¹ die oben angegebene Bedeutung hat,
reagiert und diese dann in einem inerten Lösungsmittel oder ohne weiteres Lösungsmittel mit einer Verbindung der Formel (V) 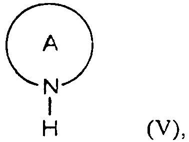 in welcher der Ring A die oben angegebene Bedeutung hat,
zu einer Verbindung der Formel (I-A) 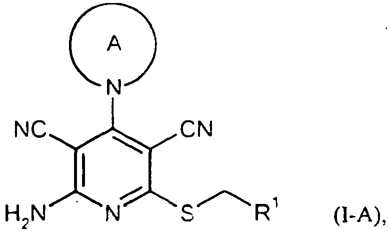 in welcher R¹ und der Ring A die zuvor angegebenen Bedeutungen haben,
umsetzt
und die Verbindungen der Formel (I-A) gegebenenfalls mit den entsprechenden (i) Lösungsmitteln und/oder (ii) Basen oder Säuren in ihre Solvate, Salze und/oder Solvate der Salze überführt.

Das zuvor beschriebene Verfahren kann durch das folgende Reaktionsschema erläutert werden: 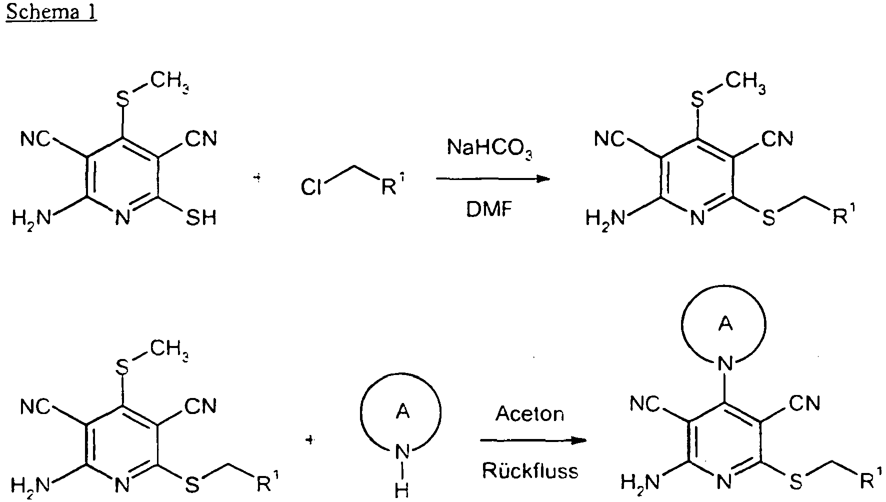 [zum zweiten Reaktionsschritt vgl. auch D. Briel et al., J. Chem. Res. Miniprint 7, 1841-1859 (1991)].

Als Lösungsmittel für den Verfahrensschritt (II) + (III) → (IV) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether. Methyl-tert.-butylether, 1,2-Dimethoxyethan. Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Bevorzugt wird Dimethylformamid als Lösungsmittel verwendet.

Als Base für den Verfahrensschritt (II) + (III) → (IV) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamm, Pyridin, 1,8-Diazabicyclo[5.4.0]undec-7-en (DBU) oder 1,5-Diazabicyclo[4.3.0]non-5-en (DBN). Bevorzugt sind Alkalicarbonate und -hydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (II), eingesetzt werden.

Die Reaktion erfolgt im Allgemeinen in einem Temperaturbereich von -78°C bis +140°C, bevorzugt im Bereich von -20°C bis +80°C, insbesondere bei 0°C bis +50°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck

Als Lösungsmittel für den Verfahrensschritt (IV) + (V) → (1-A) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan oder Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Gegebenenfalls kann die Reaktion auch vorteilhaft in Gegenwart eines Überschusses der Verbindung (V) ohne Zusatz eines weiteren Lösungsmittels durchgerührt werden. Bevorzugt wird die Umsetzung in Aceton oder N-Methylpvrrolidinon als Lösungsmittel durchgeführt.

Der Verfahrensschritt (IV) + (V) → (I-A) erfolgt im Allgemeinen in einem Temperaturbereich von 0°C bis +180°C, bevorzugt im Bereich von +20°C bis +100°C, insbesondere bei +60°C bis + 100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindung der Formel (II) lässt sich auf einfache Weise durch Umsetzung von [Bis(methyl-thio)methylen]malononitril mit Cyanothioacetamid in Gegenwart einer Base wie Triethylamin erhalten. Dieses Verfahren kann durch das folgende Reaktionsschema erläutert werden:

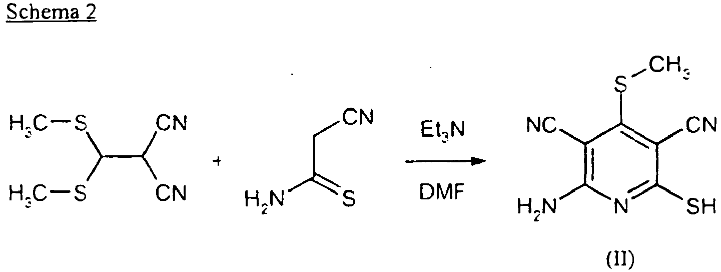

Die Verbindungen der Formel (III) sind kommerziell erhältlich, literaturbekannt oder nach literaturbekannten Methoden herstellbar. So können beispielsweise durch Reaktion von Amiden, Thioamiden bzw. Thiohamstoff-Derivaten mit einem 1,3-Dihalogenaceton substituierte Oxazol- und Thiazol-Derivate der Formel (III-A), (III-B) bzw. (III-C) erhalten werden (siehe Schema 3):

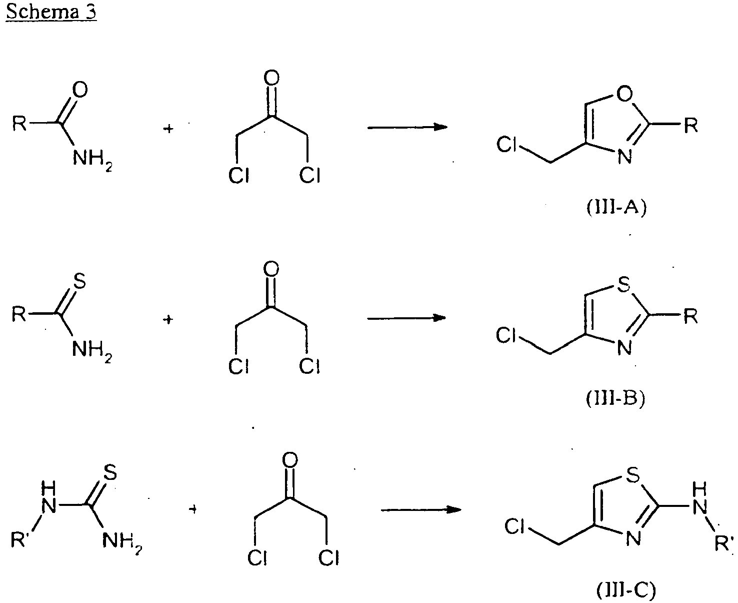

Im Falle der Verbindungen (III-C) können diese entweder analog zur Literatur hergestellt und isoliert werden [vgl. z.B. 1. Simiti et al., Chem. Ber. 95, 2672-2679 (1962)], oder sie *können in situ* erzeugt und direkt weiter mit einer Verbindung der Formel (II) umgesetzt werden. Bevorzugt ist die *in* situ-Erzeugung unter Verwendung von 1,3-Dichloraceton in Dimethylformamid oder Ethanol als Lösungsmittel. Die Darstellung erfolgt im Allgemeinen in einem Temperaturbereich von 0°C bis +140°C, bevorzugt im Bereich von +20°C bis +120°C, insbesondere bei +60°C bis +100°C.

Die Verbindungen der Formel (V) sind gleichfalls kommerziell erhältlich, literaturbekannt oder nach üblichen Methoden herstellbar [siehe z.B. A. Ladenburg, Justus Liebigs Ann. Chem. 247, 1-98 (1888); H. Nienburg, Chem. Ber. 70, 635-638 (1937); E. Koenigs, L. Neumann, Chem. Ber. 48, 956-963 (1915)].

Die erfindungsgemäßen Verbindungen der Formel (I), in welcher R² für die Gruppe -NR⁴R⁵ steht, worin mindestens einer der beiden Reste R⁴ und R⁵ nicht Wasserstoff bedeutet, können hergestellt werden, indem man Verbindungen der Formel (I-A) zunächst mit Kupfer(II)chlorid und Isoamylnitrit in einem geeigneten Lösungsmittel in Verbindungen der Formel (VI) 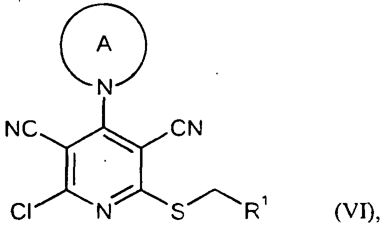 in welcher R¹ und der Ring A die zuvor angegebene Bedeutung haben,
überführt und diese anschließend mit einer Verbindung der Formel (VII) 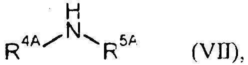 in welcher
- R^{4A}: die oben angegebene Bedeutung von R⁴ hat,
- P^{5A}: die oben angegebene Bedeutung von R⁵ hat,
jedoch mindestens einer der beiden Reste R^{4A} und R^{5A} nicht für Wasserstoff steht,
zu Verbindungen der Formel (I-B) 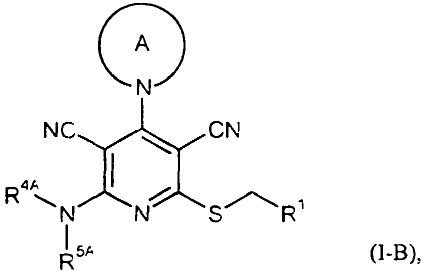 in welcher R¹, R^{4A}, R^{5A} und der Ring A jeweils die zuvor angegebenen Bedeutungen haben,
umsetzt.

Das zuvor beschriebene Verfahren kann durch das folgende Reaktionsschema erläutert werden:

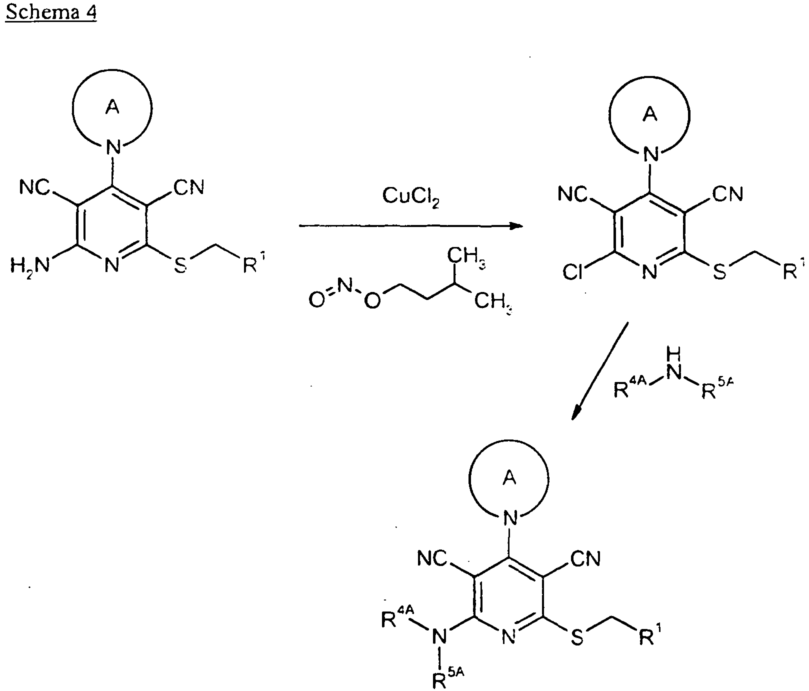

Die Umsetzung (1-A) → (VI) erfolgt im Allgemeinen in einem Molverhältnis von 2 bis 12 Mol Kupfer(II)chlorid und 2 bis 12 Mol Isoamylnitrit bezogen auf 1 Mol der Verbindung der Formel (I-A).

Als Lösungsmittel für den Verfahrensschritt (I-A) → (VI) eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische der zuvor genannten Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von 0°C bis +100°C, insbesondere bei +20°C bis +80°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Umsetzung (VI) + (VII) → (I-B) erfolgt im Allgemeinen in einem Molverhältnis von 1 bis 8 Mol der Verbindung der Formel (VII) bezogen auf 1 Mol der Verbindung der Formel (VI).

Als Lösungsmittel für den Verfahrensschritt (VI) + (VII) → (1-B) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril, Pyridin oder Dimethylsulfoxid. Wasser ist als Lösungsmittel ebenfalls geeignet. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +150°C, insbesondere bei +20°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (VII) sind entweder kommerziell erhältlich, dem Fachmann bekannt oder nach üblichen Methoden herstellbar.

Die erfindungsgemäßen Verbindungen der Formel (I), in welcher R² für Wasserstoff steht, können hergestellt werden, indem man Verbindungen der Formel (1-A) in einem geeigneten Lösungsmittel mit Isoamylnitrit in Gegenwart einer *katalyrischen* Menge Kupfer(II)chlorid umsetzt. Diese Methode kann durch das folgende Reaktionsschema erläutert werden:

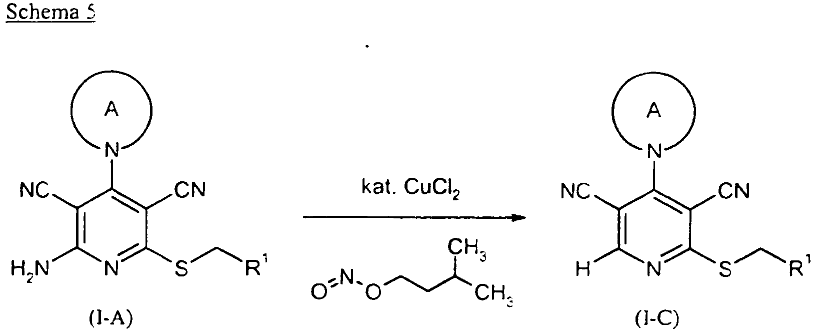

Die Umsetzung (I-A) → (I-C) erfolgt im Allgemeinen in einem Molverhältnis von 0.01 bis 0.2 Mol Kupfer(II)chlorid und 2 bis 12 Mol Isoamylnitrit bezogen auf 1 Mol der Verbindung der Formel (I-A).

Als Lösungsmittel für die Reaktion (I-A) → (I-C) eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische der genannten Lösungsmittel einzusetzen. Bevorzugte Lösungsmittel sind Tetrahydrofuran und Dimethylformamid.

Die Reaktion erfolgt im Allgemeinen in einem Temperaturbereich von -78°C bis +150°C, bevorzugt im Bereich von 0°C bis +80°C, insbesondere bei + 10°C bis +40°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die erfindungsgemäßen Verbindungen der Formel (I), in welcher R² für gegebenenfalls substituiertes (C₁-C₆)-Alkyl oder (C₁-C₆)-Alkoxy steht, sind in Analogie zu literaturbeschriebenen Methoden ausgehend von Verbindungen der Formel (VI) herstellbar [vgl. z.B. D. Mabire et al., J. Med. Chem. 48, 2134-2153 (2005)]. Alternativ können die Verbindungen der Formel (I), in welcher R² für gegebenenfalls substituiertes (C₁-C₆)-Alkoxy steht, auch durch Alkylierung von Verbindungen der Formel (VIII) erhalten werden (siehe Schema 6):

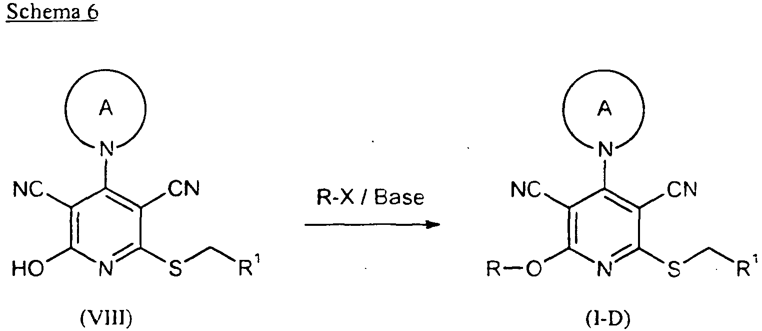

Die Verbindungen der Formel (VIII) ihrerseits sind nach literaturbekannten Methoden aus Verbindungen der Formel (VI) oder (I-A) zugänglich [vgl. z.B. G. Lavecchia et al., Tetrahedron Lett. 45, 6633-6636 (2004)].

Überraschenderweise zeigen die erfindungsgemäßen Verbindungen ein nicht vorhersehbares, wertvolles pharmakologisches Wirkspektrum und sind daher insbesondere zur Prophylaxe und/oder Behandlung von Erkrankungen geeignet. Darüber hinaus weisen die erfindungsgemäßen Substanzen gegenüber den Verbindungen aus dem Stand der Technik ein verbessertes Absorptionsverhalten im Körper und/oder eine verbesserte Löslichkeit in Wasser und anderen physiologischen Medien auf, was beispielsweise für ihre galenische Formulierbarkeit und/oder die parenterale Anwendung von Vorteil ist.

Die pharmazeutische Wirksamkeit der erfindungsgemäßen Verbindungen lässt sich durch ihre Wirkung als potente, selektive Liganden an Adenosin A1- und/oder A2b-Rezeptoren erklären. Sie wirken hierbei als selektive A1- oder als selektive duale A1/A2b-Agonisten.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung an A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Die Verbindungen der Formel (I) sind allein oder in Kombination mit einem oder mehreren anderen Wirkstoffen zur Prophylaxe und/oder Behandlung verschiedene Erkrankungen geeignet, so beispielsweise insbesondere bei Hypertonie und anderen Erkrankungen des Herzkreislauf-Systems (kardiovaskulären Erkrankungen) sowie zur Kardioprotektion.

Im Sinne der vorliegenden Erfindung sind unter Erkrankungen des Herzkreislauf-Systems bzw. kardiovaskulären Erkrankungen neben der Hypertonie beispielsweise insbesondere die folgenden Erkrankungen zu verstehen: periphere und kardiale Gefäßerkrankungen, koronare Herzerkrankung, koronare Restenose wie z.B. Restenose nach Ballondilatation von peripheren Blutgefäßen, akutes Koronarsyndrom, stabile und instabile Angina pectoris, Herzinsuffizienz, Tachykardien, Arrhythmien, Vorhof- und Kammerflimmern sowie periphere Durchblutungsstörungen.

Weiterhin eignen sich die erfindungsgemäßen Verbindungen insbesondere auch zur Reduktion des von einem Infarkt betroffenen Myokardbereichs sowie zur Prophylaxe von Sekundärinfarkten.

Des weiteren sind die erfindungsgemäßen Verbindungen insbesondere zur Prophylaxe und/oder Behandlung von thromboembolischen Erkrankungen und Ischämien wie Myokardinfarkt, Himschlag und transitorischen ischämischen Attacken sowie zur Protektion von Organen bei Transplantationen und operativen Eingriffen, beispielsweise am Herzen, geeignet.

Weitere Indikationsgebiete, für die die erfindungsgemäßen Verbindungen verwendet werden können, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandiung von inflammatorischen Erkrankungen, wie z.B. Asthma und entzündliche Dermatosen, von neuroinflammatorischen Erkrankungen des Zentralnervensystems, wie beispielsweise Zustände nach Hirninfarkt, der Alzheimer-Erkrankung, weiterhin auch von neurodegenerativen Erkrankungen sowie von Schmerzzuständen, Krebs und Übelkeit und Erbrechen in Verbindung mit Krebstherapien.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasien, zystische Fibrose (Mukoviszidose) und pulmonale Hypertonie.

Schließlich kommen die erfindungsgemäßen Verbindungen beispielsweise insbesondere auch für die Prophylaxe und/oder Behandlung von Diabetes, insbesondere Diabetes mellitus, diabetischen Folgeerkrankungen wie z.B. Nephropathie und Neuropathie, des Metabolischen Syndroms sowie von Dyslipidämien in Betracht.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten) sowie Analgetika wie beispielsweise Aspirin.

Gegenstand der vorliegenden Erfindung sind insbesondere Kombinationen mindestens einer der erfindungsgemaßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktasc-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Padikalfänger;
- Antidiabetika, die in der Roten Liste 2004/II, Kapitel 12 genannt sind, sowie beispielhaft und vorzugsweise jenen aus der Gruppe der Sulphonylhamstoffe, Biguanide, Meglitinid-Derivate, Glukosidase-Inhibitoren, Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberen-enzymen, die an der Stimulation der Glukoneogenese und/oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin All-Antagonisten, ACE-Hemmer, Renin-Inhibitoren, beta-Rezeptoren-Blocker, alpha-Rezeptoren-Blocker, Diuretika, Phosphodiesterase-Inhibitoren, sGC-Stimulatoren, Verstärker der cGMP-Spiegel, Aldosteron-Antagonisten, Mineralocorticoid-Rezeptor-Antagonisten, ECE-Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten. PPAR-γ-Agonisten, PPAR-5-Agonisten, polymeren Gallensäureadsorber, Gallensäure-Reabsorptionshemmer, Antioxidantien/Radikalfänger sowie der Cartnabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausfültrungsform der Erfindung werden die erfindungsgemäßen Verbindungen in Kombination mit einem Thyroidhormon und/oder Thyoidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfanger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Gluk-osidase-Inhiblioren und PPAR-γ-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin All-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausfühmngsform der Erfindung werden die erfindungsgemäßen Verbindungen in Kombination mit einem Angiotensin All-Antagonisten, wie beispielhaft und vorzugsweise Losanan, Valsanan, Candesanan, Embusanan, Olmesanan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carleolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorihiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibilor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Weiterer Gegenstand der vorliegenden Erfindung sind Arzneimittel, die mindestens eine erfindungsgemäße Verbindung, üblicherweise zusammen mit einem oder mehreren inenen, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisiener und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Han- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarieriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infüsionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polvoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsaneaben von flüssig/flüssig-Lösungen beziehen sich jeweils auf das Volumen.

### A. Beispiele

### Verwendete Abkürzungen:

- Bsp.: Beispiel
- DC: Dünnschichtchromatographie
- DCI: direkte chemische Ionisation (bei MS)
- DMF: N,N-Dimethylformamid
- DMSO: Dimethylsulfoxid
- d. Th.: der Theorie (bei Ausbeute)
- EE: Ethylacetat (Essigsäureethylester)
- EI: Elektronenstoß-Ionisation (bei MS)
- ESI: Elektrospray-Ionisation (bei MS)
- Et: Ethyl
- Fp.: Schmelzpunkt
- ges.: gesättigt
- h: Stunde(n)
- HPLC: Hochdruck-, Hochleistungsflüssigchromatographie
- kat.: katalytisch
- konz.: konzentriert
- LC-MS: Flüssigchromatographie-gekoppelte Massenspektrometrie
- Lit.: Literatur(stelle)
- Lsg.: Lösung
- min: Minute(n)
- MS: Massenspektrometrie
- NMR: Kernresonanzspektrometrie
- RP-HPLC: reverse phase HPLC
- RT: Raumtemperatur
- R,: Retentionszeit (bei HPLC)
- TFA: Trifluoressigsäure
- THF: Tetrahydrofuran
- verd.: verdünnt
- wässr.: wässrig

### HPLC- und LC-MS-Methoden:

### Methode 1 (HPLC):

Instrument: Hewlett Packard Series 1050; Säule: Symmetry TM C18 3.9 x 150 mm; Fluss: 1.5 ml/min; Eluent A: Wasser, Eluent B: Acetonitril: Gradient: → 0.6 min 10% B → 3.8 min 100% B → 5.0 min 100% B → 5.5 min 10% B; Stopzeit: 6.0 min; Injektionsvolumen: 10 µl; Diodenarraydetektor-Signal: 214 und 254 nm.

### Methode 2 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B →7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.

### Methode 3 (LC-MS):

Instrument: Micromass Quattro LCZ mit HPLC Agilent Serie 1100; Säule: Phenomenex Gemini 3µ 30 mm x 3.00 mm; Eluent A:11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (LC-M S):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B:11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Instrument: Micromass Quattro LCZ mit HPLC Agilent Serie 1100; Säule: Phenomenex Onyx Monolithic C18, 100 mm x 3 mm; Eluent A: 11 Wasser + 0.5 ml 50%-ige Ameisensäure, Eluent B: 11 Acetonitril + 0.5 mm 50%-ige Ameisensäure; Gradient: 0.0 min 90% A → 2 min 65% A → 4.5 min 5% A → 6 min 5% A; Fluss: 2 ml/min, Ofen: 40°C; UV-Detektion: 208-400 nm.

### Methode 6 (präparative HPLC):

Gerätetyp HPLC: Abimed/Gilson Pump 305/306; Manometric Module 806; UV Knauer Variable Wavelength Monitor; Säule: Gromsil C18, 10 nm, 250 mm x 30 mm; Eluent A: 1 1 Wasser + 0.5 ml 99% TFA, Eluent B: 1 1 Acetonitril; Gradient: 0.0 min 2% B → 10 min 2% B → 50 min 90% B; Fluss: 20 ml/min; Volumen: 628 ml A und 372 ml B.

### Methode 7 (LC-MS):

Gerätetyp MS: Waters ZQ; Gerätetyp HPLC: Waters Alliance 2795; Säule: Merck Chromolith RP18e, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C: UV-Detektion: 210 nm.

### Methode 8 (LC-MS):

Gerätetyp MS: Micromass ZQ; Gerätetyp HPLC: Waters Alliance 2795; Säule: Phenomenex Synergi 2.5µ MAX-RP 100A Mercury, 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A →4.0 min 5% A → 4.01 min 90% A; Fluss: 2 ml/min; Ofen: 50°C; UV-Detektion: 210 nm.

### Methode 9 (LC-MS):

Instrument: Micromass QuattroPremier mit Waters UPLC Acquity; Säule: Thermo Hypersil GOLD 1.9µ, 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Ofen: 50°C; Fluss: 0.33 ml/min; UV-Delektion: 210 nm.

### Ausgangsverbindungen und Intermediate:

### Beispiel 1A

### 2-Amino-6-mercapto-4-(methylthio)-pyridin-3,5-dicarbonitril

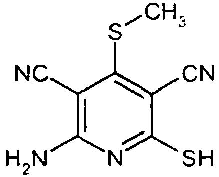

10 g (58.74 mmol) [Bis(methylthio)methylen]malononitril und 6.5 g (64.61 mmol) Cyanothioacetamid werden in 20 ml DMF vorgelegt und bei Raumtemperatur tropfenweise mit 16.4 ml (117.5 mmol) Triethylamin versetzt. Der Ansatz wird 8 h bei Raumtemperatur gerührt und für weitere zwei Tage stehengelassen. Das Gemisch wird danach auf 250 ml 3 N Salzsäure gegeben. Der ausfallende Niederschlag wird abgesaugt, mit Wasser und Aceton gewaschen und getrocknet. Es resultiert ein gelbes Pulver
Ausbeute: 12.9 g (99% d. Th.)
¹H-NMR (400 MHz, CDCl₃): δ = 3.98 (s, 1H), 2.72 (s, 3H).
LC-MS (Methode 3): R₁ = 1.43 min; MS (ESIpos): m/z = 222 [M+H]⁻.

### Beispiel 2A

### 2-Amino-6-({(2-(4-chlorphenyl)-1,3-thiazol-4-yl)methyl}thio)-4-(methylthio)-pyidin-3,5-dicarbonitril

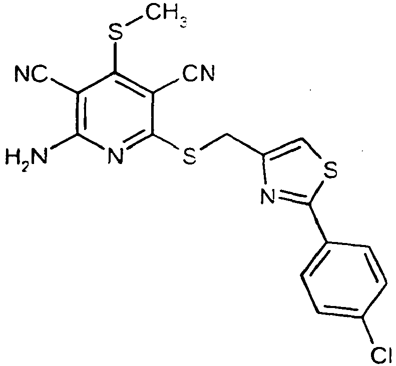

250 mg (1.13 mmol) 2-Amino-6-mercapto-4-(methylthio)-pyridin-3,5-dicarbonitril, 412 mg (1.69 mmol) 4-(Chlormethyl)-2-(4-chlorphenyl)-1,3-thiazol und 378 mg (4.50 mmol) Natriumhydrogencarbonat werden in 5 ml DMF zusammengegeben und 12 h bei Raumtemperatur gerührt. Es fällt ein voluminöser Feststoff aus, welcher über eine Glasfritte abgesaugt, dreimal mit Wasser sowie zweimal mit Diethylether gewaschen und getrocknet wird. Es resultiert ein weißes Pulver.
Ausbeute: 474 mg (98% d. Th.)
¹H-NMR (400 MHz, CDCl₃): δ = 8.13 (br. s, 2H), 7.93 (d, 2H), 7.87 (s, 1H), 7.55 (d, 2H), 4.59 (s, 2H), 2.72 (s, 3H).
LC-MS (Methode 2): Rₜ = 2.72 min; MS (ESIpos): m/z = 430 [M+H]⁺.

### Beispiel 3A

### 2-Amino-6-[({2-[(4-fluorphenyl)amino]-1,3-thiazol-4-yl}methyl)thio]-4-(methylthio)-pyridin-3,5-dicarbonitril

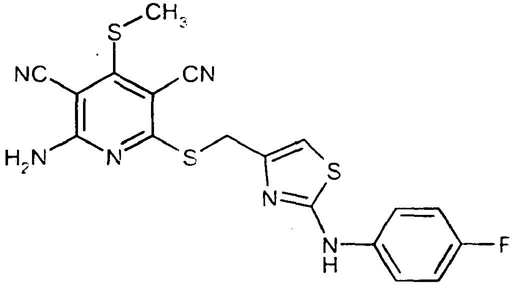

Beispiel 3A wird in Analogie zu Beispiel 2A aus 2-Amino-6-mercapto-4-(methylthio)-pyridin-3,5-dicarbonitril und 4-(Chlormethyl)-*N*-(4-fluorphenyl)-1,3-thiazol-2-amin-Hydrochlorid, welches *in situ* aus 4-Fluorphenylthiohamstoff und 1,3-Dichloraceton erzeugt wird, hergestellt (vgl. WO 2006/027142, Beispiel 5A).
Ausbeute: 95% d. Th.
¹H-NMR (400 MHz, CDCl₃): δ = 10.21 (s, 1H), 8.10 (br. s, 2H), 7.55-7.63 (m, 2H), 7.08-7.14 (m, 2H), 6.91 (s, 1H), 4.42 (s, 2H), 3.73 (s, 3H).
LC-MS (Methode 3): Rₜ = 2.54 min; MS (ESIpos): m/z = 429 [M+H]⁺.

### Beispiel 4A

### 2-Chlor-6-({[2-(4-chlorphenyl)-1,3-thiazol-4-yl]methyl}thio)-4-(piperidin-1-yl)-pyridin-3,5-dicarbonitril

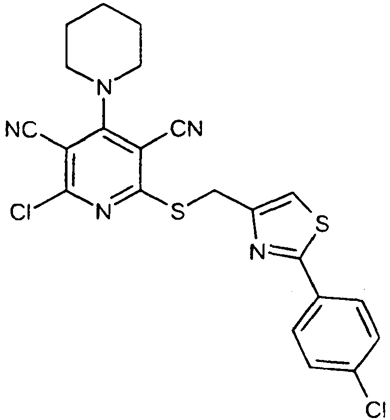

2.30 g (4.93 mmol) der Verbindung aus Beispiel 2 werden zu einer Suspension von 3.85 g (29.55 mmol) Isopentylnitrit und 3.97 g (29.55 mmol) Kupfer(II)chlorid in 40 ml trockenem Acetonitril gegeben und der Ansatz 3 h bei +60°C gerührt. Zur Reaktionslösung werden dann 20 ml 1 N Salzsäure gegeben. Die wässrige Phase wird zweimal mit je 30 ml Essigsäureethylester extrahiert. Die vereinigten organischen Phasen werden einmal mit 10 ml gesättigter Natriumhydrogencarbonat-Lösung und einmal mit 10 ml gesättigter Natriumchlorid-Lösung gewaschen und über Magnesiumsulfat getrocknet. Nach Entfernen des Lösungsmittels am Rotationsverdampfer wird das Rohprodukt an Kieselgel 60 chromatographisch gereinigt (Laufmittel: Gradient Cyclohexan/Essigsäureethyl ester 10:1 → 1:4).
Ausbeute: 1.50 g (59% d. Th.)
¹H-NMR (400 MHz, DMSO-d₆): δ = 7.94 (d, 2H), 7.68 (s, 1H), 7.57 (d, 2H), 4.63 (s, 2H), 3.68-3.58 (br. s, 4H), 1.72-1.58 (br. s, 6H).
LC-MS (Methode 2): Rₜ = 3.23 min; MS (ESIpos): m/z = 486 [M+H]⁺.

Die in der folgenden Tabelle aufgeführten Verbindungen werden analog zu Beispiel 2A aus den entsprechenden Ausgangsverbindungen hergestellt:

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **5A** | 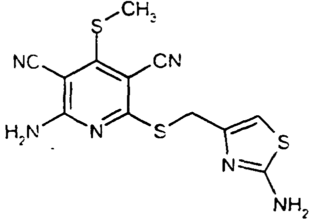 | 1.87 min (7): m/z = 335 | 8.28-7.93 (br. s, 1H), 6.97 (s, 2H), 6.59 (s, 1H), 4.27 (s, 2H), 2.73 (s, 3H). |
| | (96% d. Th.) | | |
| **6A** | 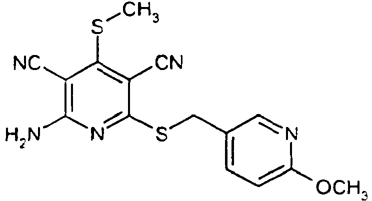 | 3.43 min (5), m/2 = 344 | 8.35 (d, 1H), 8.28-7.88 (br. s, 2H), 7.81 (dd, 1H), 6.77 (d, 1H), 4.49 (s, 2H), 3.81 (s, 3H), 2.72 (s, 3H). |
| | (90% d. Th.) | | |
| **7A** | 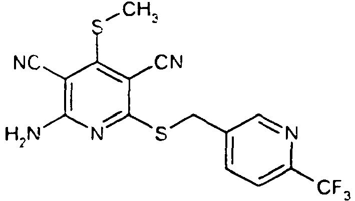 | 3.63 min (5), m/z = 382 | 8.97 (d, 1H), 8.48-7.99 (br. s, 2H), 8.22 (dd, 1H), 7.84 (d, 1H), 4.54 (s, 2H), 2.72 (s, 3H). |
| | (90% d. Th.) | | |
| **8A** | 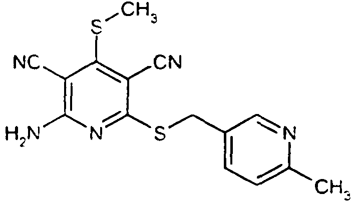 | 1.96 min (5); m/z=328 | 8.60 (d, 1H), 8.47-7.86 (br. s, 2H), 7.79 (dd, 1H), 7.18 (d, 1 H), 4.41 (s. 2H), 2.72 (s, 3H), 2.42 (s, 3H). |
| | (84% d. Th.) | | |

| **Beispiel Nr.** | **Struktur (Ausbeute)** | **LC-MS: Rₜ [min] (Methode); MS(ESI): m/z [M+H]⁺** | **¹H-NMR (DMSO-d₆): δ =** |
|---|---|---|---|
| **9A** | 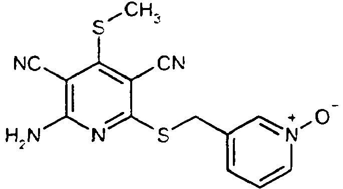 | 2.15 min (7); m/z = 330 | |
| | (90% d. Th.) | | |

### Beispiel 10A

### 2-({[2-(4-Chlorphenyl)-1,3-thiazol-4-yl]methyl}thio)-6-hydroxy-4-(piperidin-1-yl)-pyridin-3,5-dicarbonitril

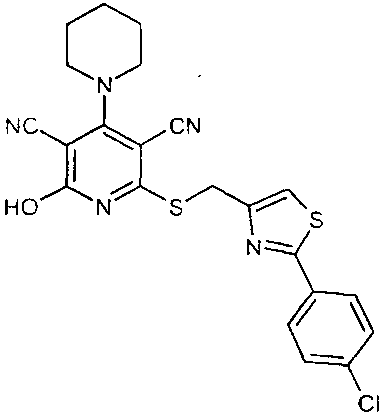

400 mg (0.82 mmol) der Verbindung aus Beispiel 4A werden in 8 ml trockenem DMF vorgelegt. Nach Zugabe von 110 mg (0.99 mmol) Kalium-tert.-butylat wird 30 min bei RT gerührt. Anschließend wird eine Lösung von 74 mg (0.82 mmol) Glykolsäuremethylester in 1 ml trockenem DMF zugetropft und die Reaktionsmischung 20 h bei RT gerührt. Das Lösungsmittel wird danach am Rotationsverdampfer entfernt und der Rückstand direkt über präparative HPLC gereinigt (Säule: YMC GEL ODS-AQ S-5 / 15 µm; Laufmittelgradient: Acetonitril/Wasser 10:90 → 95:5).
Ausbeute: 45 mg (20% d. Th.)
¹H-NMR (400 MHz, DMSO-d₆): δ = 7.97 (d, 2H), 7.72 (s, 1H), 7.57 (d, 2H), 4.64 (s, 2H), 3.54-3.44 (br. s, 4H), 1.68-1.56 (br. s, 6H).
LC-MS (Methode 5): R, = 4.18 min; MS (ESIpos): m/z = 468 [M+H]⁺.

### Beispiel 11A

### 2-Amino-6-({[2-(4-chlorphenyl)-1,3-oxazol-4-yl]methyl}sulfanyl)-4-(methylsulfanyl)-pyridin-3,5-dicarbonitril

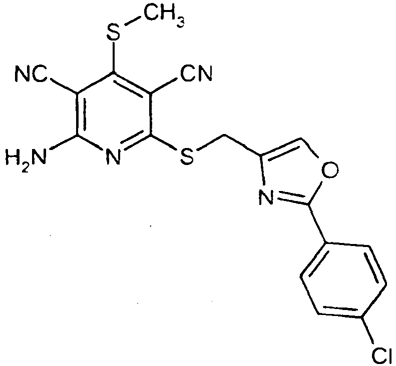

Die Titelverbindung wird analog zu Beispiel 2A aus den entsprechenden Edukten hergestellt.
Ausbeute: 362 mg (88% d. Th.)
LC-MS (Methode 8): Rₜ = 2.27 min; MS (ESIpos): m/z = 414 [M+H]⁺.

### Beispiel 12A

### 2-Amino-6-{[(6-chlor-1-oxidopyridin-3-yl)methyl]sulfanyl}-4-(methylsulfanyl)-pyridin-3,5-dicarbonitril

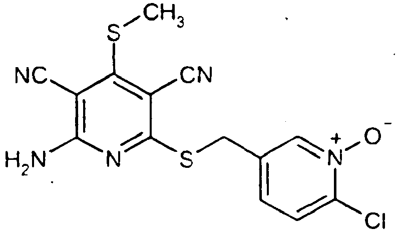

Die Titelverbindung wird analog zu Beispiel 2A aus den entsprechenden Edukten hergestellt. Die Ausgangsverbindung 2-Chlor-5-(chlormethyl)pyridin-1-oxid ist nach einer Literaturvorschrift erhältlich [J.W. Tilley, P. Levitan, R.W. Kierstaed, J. Heterocycl. Chem. 1979, 16, 333-337].
Ausbeute: 267 mg (54% d. Th.)
¹H-NMR (400 MHz, CDCl₃): δ = 8.73 (d, 1H), 8.12-7.85 (br. s, 2H), 7.72 (d, 1H), 7.49 (dd, 1H), 4.38 (s, 2H), 2.72 (s, 3H).
LC-MS (Methode 5): Rₜ = 2.67 min; MS (ESIpos): m/z = 364 [M+H]⁺.

### Ausführungsbeispiele:

### Beispiel 1

### 2-Amino-6-({[2-(4-chlorphenyl)-1,3-thiazol-4-yl]methyl}thio)-4-(4-hydroxypiperidin-1-yl)-pyridin-3,5-dicarbonitril

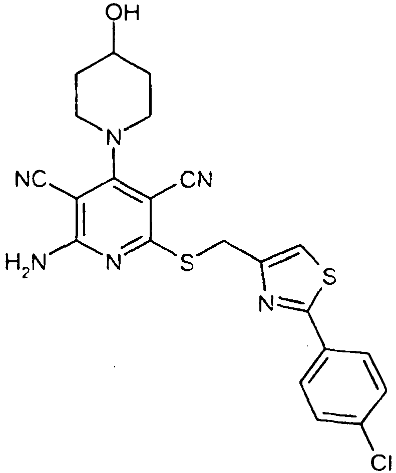

100 mg (0.23 mmol) 2-Amino-6-({[2-(4-chlorphenyl)-1,3-thiazol-4-yl]methyl}thio)-4-(methylthio)-pyridin-3,5-dicarbonitril und 1000 mg (9.89 mmol) 4-Hydroxypiperidin werden in 2 ml Aceton zusammengegeben und 8 h unter Rückfluss gerührt. Der Ansatz wird dann auf eine Mischung aus halbkonzentrierter wässriger Ammoniumchlorid-Lösung und Ethylacetat gegeben. Nach kräftiger Durchmischung wird die organische Phase abgetrennt, mit gesättigter Natriumchlorid-Lösung gewaschen, über Magnesiumsulfat getrocknet, über eine Glasfritte abgesaugt und das Lösungsmittel am Rotationsverdampfer entfernt. Der Rückstand wird zunächst mittels Säulenchromatographie an Kieselgel 60 (Laufmittel: Gradient Toluol/Ethylacetat 3:1 → 1:1), dann mittels präparativer HPLC gereinigt (Säule: YMC GEL ODS-AQ S-5 / 15 µm; Laufmittelgradient: Acetonitril/Wasser 10:90 → 95:5). Die Titelverbindung wird als weißes Pulver erhalten.
Ausbeute: 40 mg (36% d. Th.)
¹H-NMR (400 MHz, DMSO-d₆): δ = 7.93 (d, 2H), 7.82 (s, 1H), 7.67 (br. s, 2H), 7.56 (d, 2H), 4.54 (s, 2H), 3.68-3.75 (m, 3H), 3.25-3.35 (m, 2H), 1.80-1.88 (m, 2H), 1.44-1.57 (m, 2H).
LC-MS (Methode 2): R, = 2.38 min; MS (ESIpos): m/z = 483 [M+H]⁺.

Die in der folgenden Tabelle aufgeführten Verbindungen werden analog zu Beispiel 1 aus den entsprechenden Ausgangsverbindungen hergestellt:

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **2** | 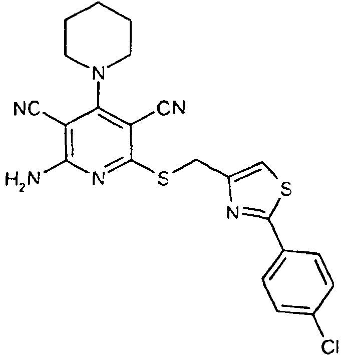 | 2.95 min (2); m/z = 467 | |
| | (53% d. Th.) | | |
| **3** | 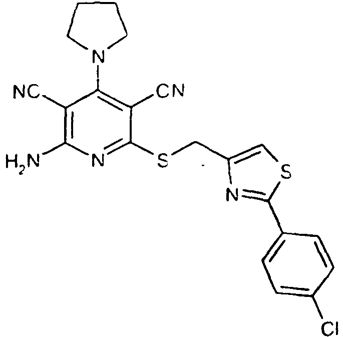 | 3.06 min (4); m/z = 453 | 7.92 (d, 2H), 7.79 (s, 1H), 7.58 (d, 2H), 7.39 (br. s. 2H), 4.51 (s, 2H), 3.80-3.88 (m, 4H), 1.84-1.88 (m, 4H). |
| | (41 % d. Th.) | | |

| **Beispiel Nr.** | **Struktur (Ausbeute)** | **LC-MS: Rₜ [min]) (Methode); MS (ESI): m/z [M+H]⁺** | **¹H-NMR (DMSO-d₆): δ =** |
|---|---|---|---|
| **4** | 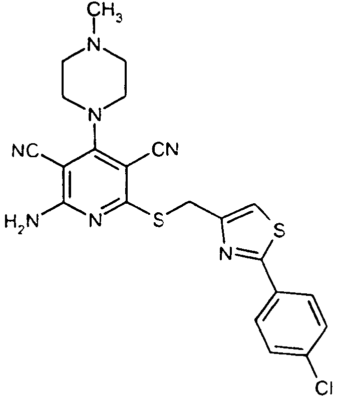 | 1.64 min (3); m/z = 482 | 7.92 (d, 2H), 7.82 (s, 1H), 7.72 (br. s, 2H), 7.56 (d, 2H), 4.54 (s, 2H), 3.49-3.56 (m, 4H), 2.38-2.48 (m, 4H), 2.20 (s, 3H). |
| | (40% d. Th.) | | |
| **5** | 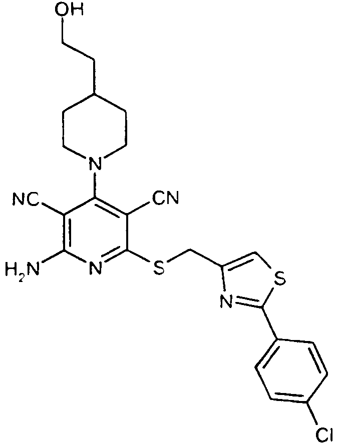 | 2.54 min (2); m/z = 51] | |
| | (37% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **6** | 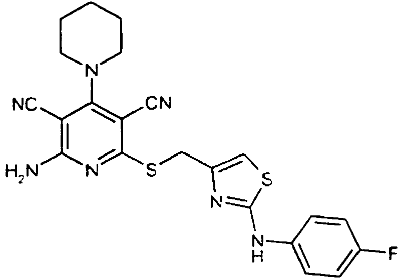 | 2.89 min (4); m/z = 466 | |
| | (97% d. Th.) | | |
| **7** | 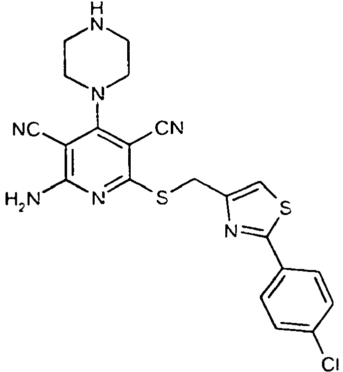 | 1.67 min (2); m/z = 468 | 7.92 (d, 2H), 7.82 (s, 1H), 7.70 (br. s, 2H), 7.56 (d, 2H), 4.56 (s, 2H), 3.40-3.46 (m, 4H), 2.74-2.80 (m, 4H). |
| | (18% d. Th.) | | |
| **8** | 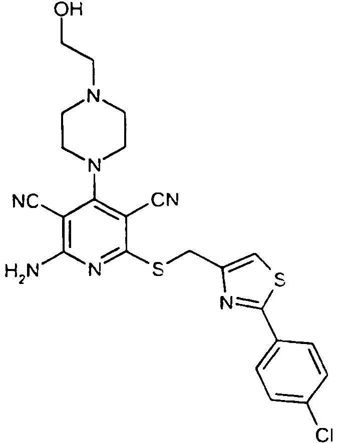 | 1.63 min (2). m/z=512 2 | |
| | (43% d. Th.) | | |
| **9** | 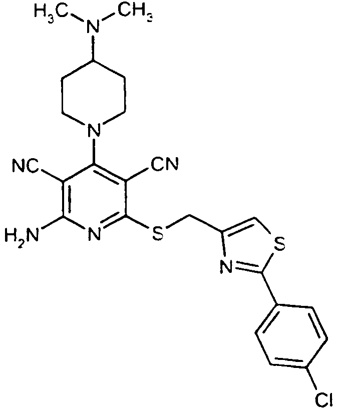 | 1.70 min (3); m/z=510 | |
| | (41% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆): δ** = x |
|---|---|---|---|
| **10** | 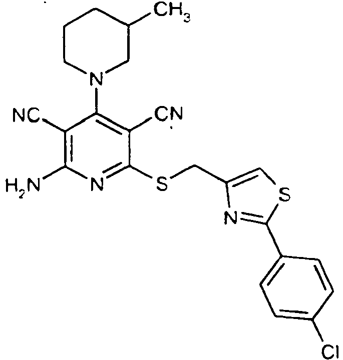 | 3.26 min (4); m/z = 481 | |
| | (52% d. Th.) | | |
| **11** | 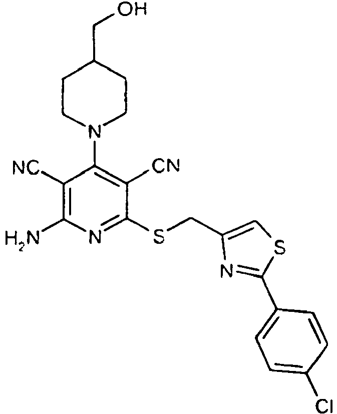 | 2.45 min (2); m/z = 497 | |
| | (15% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆): δ** |
|---|---|---|---|
| **12** | 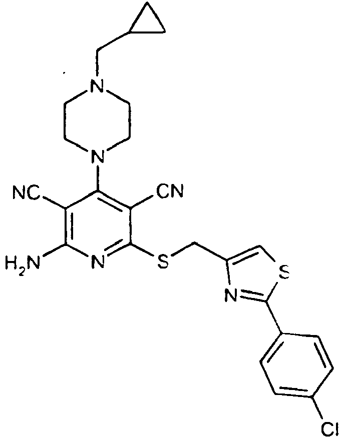 | 1.78 min (3); m/z = 522 | |
| | (17% d. Th.) | | |
| **13** | 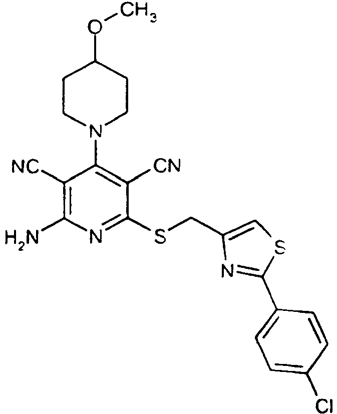 | 2.87 min (3); m/z = 497 | 7.93 (d, 2H), 7.82 (s, 1H), 7.80-7.60 (br. s, 2H), 7.56 (d, 2H), 4.54 (s, 2H), 3.71-3.60 (m, 2H), 3.48-3.39 (m, 1H), 3.38-3.29 (m, 2H), 3.31 (s, 3H). 1.99-1.88 (m, 2H), 1.62-1.51 (m, 2H). |
| | (26% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **14** | 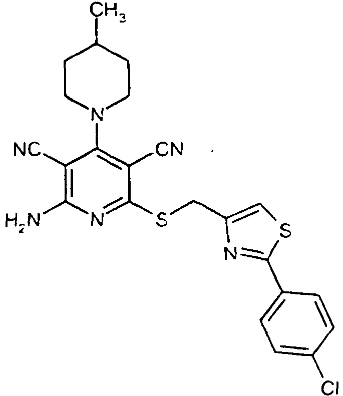 | 3.07 min (2); m/z = 481 | |
| | (40% d. Th.) | | |
| **15** | 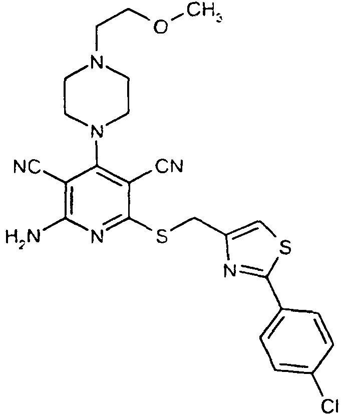 | 1.72 min (2); m/z = 526 | |
| | (35% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆):δ=** |
|---|---|---|---|
| **16** | 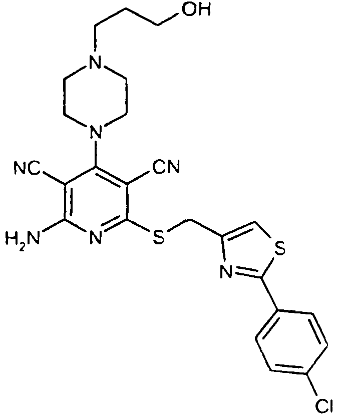 | 1.80 min (4); m/z = 526 | |
| | (30% d. Th.) | | |
| **17** | 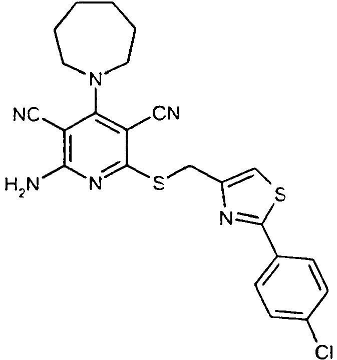 | 3.20 min (4); m/z = 481 | |
| | (12% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **18** | 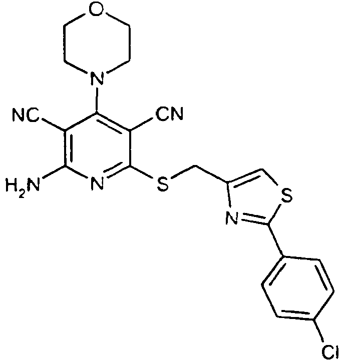 | 3.96 min (5); m/z = 469 | 7.93 (d, 2H), 7.89-7.63 (br. s, 2H), 7.83 (s, 1H), 7.55 (d, 2H), 4.54 (s, 2H), 3.73-3.66 (m, 4H), 3.58-3.51 (m, 4H). |
| | (31% d. Th.) | | |
| **19** | 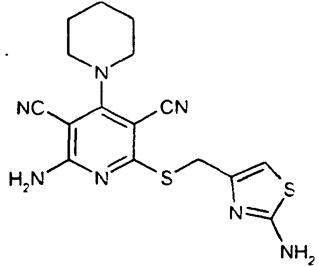 | 2.27 min (7); m/z = 372 | 7.75-7.48 (br. s, 2H), 6.95 (s, 2H), 6.54 (s, 1 H), 4.22 (s, 2H), 3.52-3.42 (br. s, 4H), 1.68-1.58 (br. s, 6H). |
| | (69% d. Th.) | | |
| **20** | 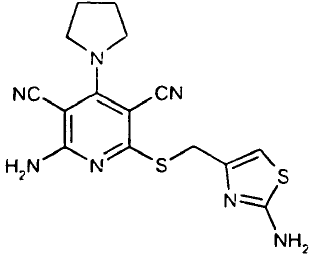 | 1.58 min (4); m/z = 358 | 7.49-7.18 (br. s, 2H), 6.94 (s, 2H), 6.54 (s, 1 H), 4.20 (s, 2H), 3.93-3.76 (br. s, 4H), 1.97-1.80 (br. s, 4H). |
| | (28% d. Th.) | | |

| **Beispiel Nr.** | **Struktur (Ausbeute)** | **LC-MS: R, [min] (Methode); MS (ESI): m/z [M+H]⁺** | **¹H-NMR (DMSO-d₆): δ =** |
|---|---|---|---|
| **21** | 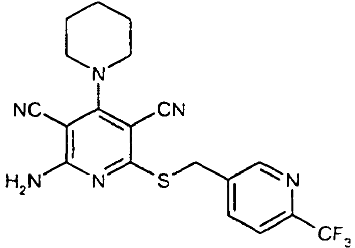 | 3.91 min (5); m/z = 419 | 8.95 (s, 1H), 8.20 (dd, 1H), 7.88-7.59 (br. s, 2H), 7.83 (d, 1H), 4.48 (s, 2H), 3.52-3.42 (br. s, 4H), 1.65-1.57 (br. s, 6H). |
| | (42% d. Th.) | | |
| **22** | 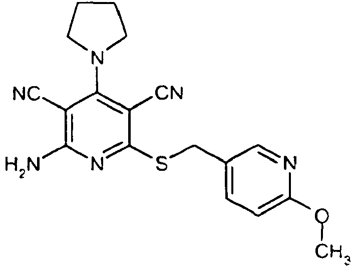 | 2.40 min (4); m/z = 367 | 8.30 (d, 1H), 7.77 (dd, 1 H), 7.53-7.39 (br. s, 2H), 6.76 (d, 1H). 4.31 (s, 2H), 3.87-3.79 (m, 4H), 1.91-1.83 (m, 4H). |
| | (13% d. Th.) | | |
| **23** | 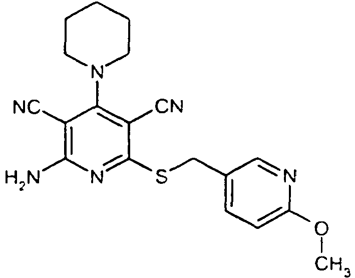 | 3.70 min (5): m/z = 381 | 8.30 (d, 1H), 7.78 (dd, 1H), 7.75-7.58 (br. s, 2H), 6.75 (d, 1H), 4.34 (s, 2H), 3.81 (s, 3H), 3.50-3.43 (br. s, 4H), 1.67-1.58 (br. s, 6H). |
| | (37% d. Th.) | | |

| **Beispiel Nr.** | **Struktur (Ausbeute)** | **LC-MS: Rₜ [min] (Methode); MS (ESI)_{:} m/z [M+H]⁺** | **¹H-NMR (DMSO-d₆):δ =** |
|---|---|---|---|
| **24** | 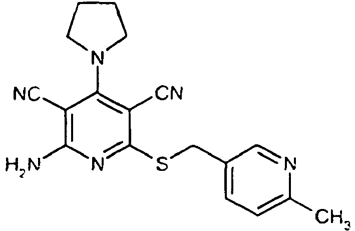 | 2.01 min (5): m/z = 351 | 8.57 (s, 1H), 7.73 (d, 1H), 7.58-7.29 (br. s, 2H), 7.16 (d, 1H), 4.35 (s, 2H), 3.88-3.77 (br. s, 4H), 1.93-1.82 (br. s, 4H). |
| | (26% d. Th.) | | |
| **25** | 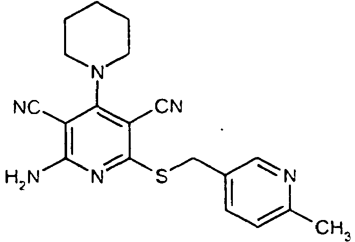 | 2.38 min (S); m/z = 365 | 8.55 (d, 1H), 7.79-7.52 (br. s, 2H), 7.74 (dd. 1H), 7.17 (d, 1H), 4.37 (s, 2H), 3.51-3.42 (br. s, 4H), 1.67-1.54 (br. s, 6H). |
| | (55% d. Th.) | | |
| **26** | 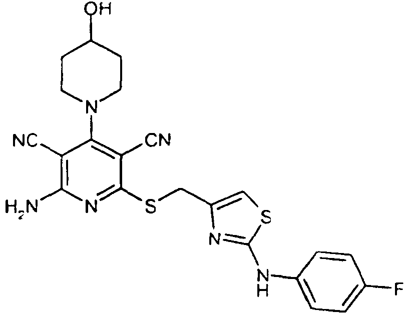 | 2.05 min (2); m/z = 482 | |
| | (11% d. Th.) | | |

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **27** | 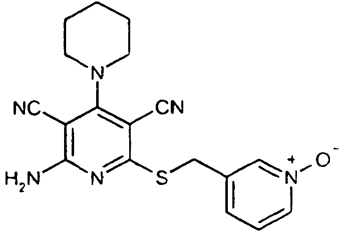 | 2.00 min (4); m/z = 367 | 8.46 (s, 1H), 8.08 (d, 1 H), 7.99-7.53 (br. s, 2H), 7.44 (d, 1H), 7.33 (pseudo-t, 1H), 4.35 (s, 2H), 3.53-3.43 (br. s, 4H), 1.68-1.57 (br. s, 6H). |
| | (30% d. Th.) | | |
| **28** | 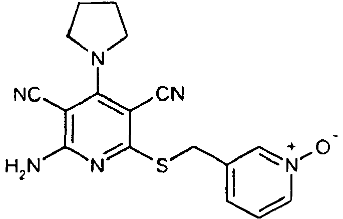 | 1.81 min (4), m/z = 353 | 8.45 (s, 1H), 8.07 (d, 1 H), 7.76-7.22 (br. s, 2H), 7.43 (d, 1H), 7.33 (pseudo-t, 1H), 4.32 (s, 2H), 3.87-3.80 (br. s, 4H), 1.92-1.83 (br. s, 4H). |
| | (57% d. Th.) | | |

### Beispiel 29

### N-[6-({[2-(4-Chlorphenyl)-1,3-thiazol-4-yl]methyl}thio)-3,5-dicyano-4-(piperidin-1-yl)-pyridin-2-yl]-beta-alamin

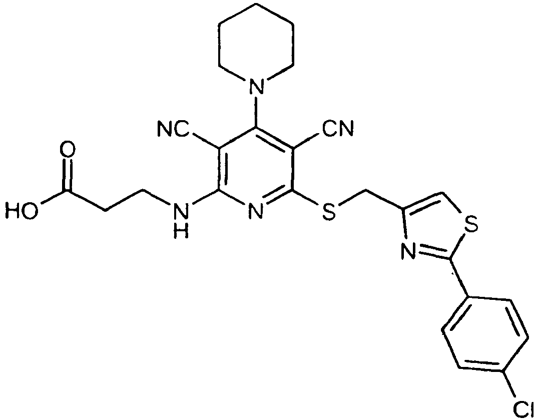

Eine Lösung von 70 mg (0.14 mmol) der Verbindung aus Beispiel 4A und 27 mg (0.30 mmol) beta-Alanin in 2 ml DMF wird 8 h bei RT gerührt. Anschließend wird 2 h bei +90°C gerührt. Nach Abkühlen auf RT wird das Reaktionsgemisch direkt über präparative HPLC gereinigt (Säule: YMC GEL ODS-AQ S-5, 15 µm; Laufmittelgradient: Acetonitril/Wasser 10:90 → 95:5).
Ausbeute: 42 mg (57% d. Th.)
¹H-NMR (400 MHz, DMSO-d₆): δ = 12.26 (s, 1H), 7.93 (d, 2H), 7.71 (t, 1H), 7.62 (s, 1H), 7.56 (d, 2H), 4.62 (s, 2H), 3.72-3.63 (m, 2H), 3.53-3.43 (m, 4H), 3.36-3.25 (m, 2H), 1.68-1.57 (br. s, 6H).
LC-MS (Methode 5): R, = 4.28 min; MS (ESIpos): m/z = 539 [M+H]⁻.

Die in der folgenden Tabelle aufgeführten Verbindungen werden analog zu Beispiel 29 aus den entsprechenden Ausgangsverbindungen hergestellt:

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **30** | 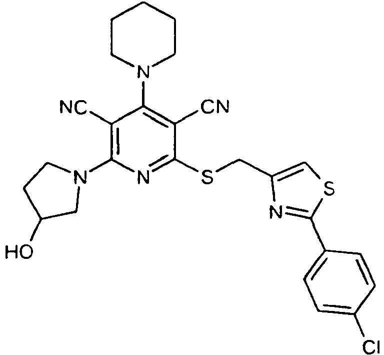 | 2.89 min (2); m/z = 537 | 10.25 (s, 1H), 8.19 (br. s, 2H), 8.35 (d. 1H), 7.92 (dd, 1H), 7.68-7.58 (m, 2H), 7.19-7.08 (m, 2H), 7.11-7.07 (m, 2H), 4.89 (t, 1H), 4.45 (s, 2H), 4.38-4.30 (m, 2H), 3.78-3.68 (m, 2H). |
| | (78% d. Th.) | | |
| **31** | 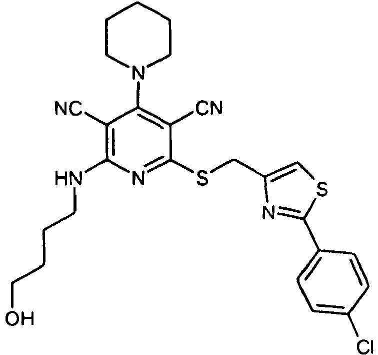 | 4.40 min (5); m/z = 539 | 7.92 (d, 2H), 7.78 (t, 1H), 7.58 (s, 1H), 7.56 (d, 2H), 4.60 (s, 2H), 4.37 (t, 1H), 3.53-3.43 (br. s, 4H), 3.42-3.36 (m, 2H), 3.33-3.27 (m, 2H), 1.70-1.57 (br. s, 6H), 1.53-1.43 (m, 2H), 1.40-1.31 (m, 2H). |
| | (88% d. Th.) | | |
| **32** | 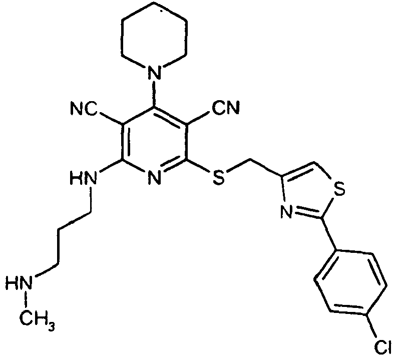 | 2.00 min (4); m/z=538 | |
| | (27% d. Th.) | | |

| **Beispiel Nr.** | **Struktur (Ausbeute)** | **LC-MS: Rₜ [min] (Methode); MS (ESI): m/z [M+H]⁺** | **¹H-NMR (DM SO-d₆): δ =** |
|---|---|---|---|
| **33** | 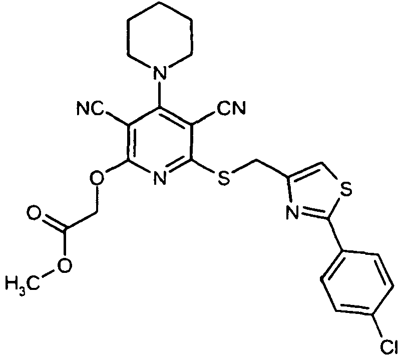 | 3.29 min (4); m/z = 540 | |
| | (9% d. Th.) | | |

### Beispiel 34

### 2-({[2-(4-Chlorphenyl)-1,3-thiazol-4-yl]methyl}thio)-4-(piperidin-1-yl)-pyridin-3,5-dicarbonitril

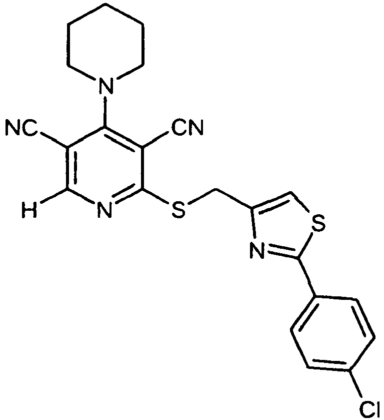

83.0 mg (0.16 mmol) der Verbindung aus Beispiel 2 werden in 4 ml trockenem THF vorgelegt. Zu dieser Lösung werden 130.7 mg (1.12 mmol) Isopentylnitrit und 2.21 mg (0.02 mmol) Kupfer(II)-chlorid gegeben und der Ansatz 20 h bei RT gerührt. Das Reaktionsgemisch wird danach direkt über präparative HPLC (Säule: YMC GEL ODS-AQ S-5, 15 µm; Laufmittelgradient: Acetonitril/Wasser 10:90 → 95:5) gereinigt.
Ausbeute: 20.0 mg (27% d. Th.)
¹H-NMR (400 MHz, DMSO-d₆): δ = 8.69 (s, 1H), 7.94 (d, 2H), 7.68 (s, 1H), 7.57 (d, 2H), 4.68 (s, 2H), 3.67-3.60 (br. s, 4H), 1.73-1.59 (br. s, 6H).
LC-MS (Methode 2): Rₜ = 3.14 min; MS (ESIpos): m/z = 452 [M+H]⁺.

### Beispiel 35

### 2-( {[2-(4-Chlorphenyl)-1,3-thiazol-4-yl]methyl}thio)-6-methoxy-4-(piperidin-1-yl)-pyridin-3,5-dicarbonitril

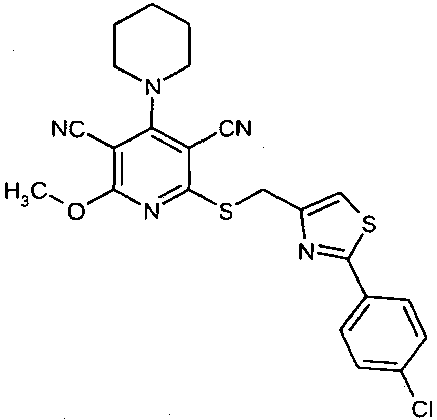

100.0 mg (0.21 mmol) der Verbindung aus Beispiel 4A werden in 2 ml trockenem Methanol vorgelegt. Zu dieser Lösung werden 12.2 mg (0.23 mmol) Natriummethylat gegeben und der Ansatz 20 h bei +65°C gerührt. Nach Abkühlen auf RT wird das Reaktionsgemisch in 2 ml Wasser eingerührt und 1 h bei RT nachgerührt. Der entstandene Niederschlag wird abgesaugt und mit 1 ml kaltem Wasser gewaschen. Die Reinigung erfolgt mittels präparativer HPLC (Säule: YMC GEL ODS-AQ S-5, 15 µm; Laufmittelgradient: Acetonitril/Wasser 10:90 → 95:5).
Ausbeute: 14.0 mg (14% d. Th.)
¹H-NMR (400 MHz, DMSO-d₆): δ = 7.96-7.91 (m, 2H), 7.68 (s, 1H), 7.60-7.54 (m, 2H), 4.70 (s, 2H), 3.62-3.53 (br. s, 4H), 1.68-1.48 (br. s, 6H).
LC-MS (Methode 7): Rₜ = 4.58 min; MS (ESIpos): m/z = 482 [M+H]⁺.

### Beispiel 36

### Ethyl-{[6-({[2-(4-chlorphenyl)-1,3-thiazol-4-yl]methyl}thio)-3,5-dicyano-4-(piperidin-1-yl)-pyridin-2-yl]oxy} acetat

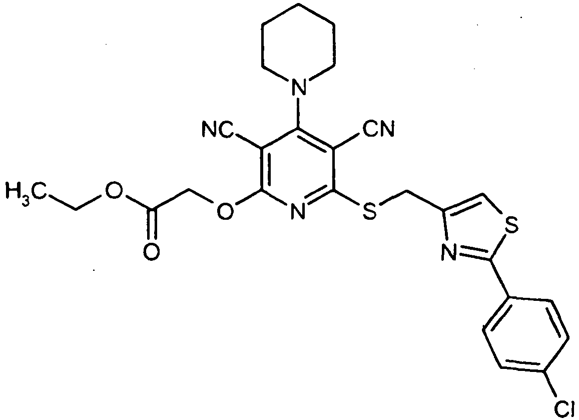

43 mg Iodessigsäureethylester werden in 2.4 ml trockenem Toluol vorgelegt. Anschließend werden 47 mg (0.10 mmol) der Verbindung aus Beispiel 10A sowie 14 mg (0.05 mmol) Silbercarbonat zugegeben. Das Reaktionsgemisch wird 24 h unter Lichtausschluss gerührt. Der Ansatz wird danach mit 1 ml Ethylacetat verdünnt und mit 1 ml ges. wässr. Natriumhydrogencarbonat-Lösung versetzt. Nach Trennen der Phasen wird die organische Phase über Magnesiumsulfat getrocknet. Das Lösungsmittel wird am Rotationsverdampfer entfernt und der Rückstand über präparative HPLC gereinigt (Säule: YMC GEL ODS-AQ S-5 / 15 µm; Laufmittelgradient: Acetonitril/Wasser 10:90 → 95:5).
Ausbeute: 35 mg (63% d. Th.)
¹H-NMR (400 MHz, DMSO-d₆): δ = 7.93 (d, 2H), 7.74 (s, 1H), 7.57 (d, 2H), 5.12 (s, 2H), 4.58 (s, 2H), 4.11 (q, 2H), 3.66-3.57 (br. s, 4H), 1.71-1.61 (br. s, 6H), 1.12 (t, 3H).
LC-MS (Methode 7): R, = 4.51 min; MS (ESIpos): m/z = 554 [M+H]⁺.

### Beispiel 37

### 2-Amino-6-({[2-(4-chlorphenyl)-1,3-oxazol-4-yl]methyl}sulfanyl)-4-(piperidin-1-yl)-pyridin-3,5-dicarbonitril

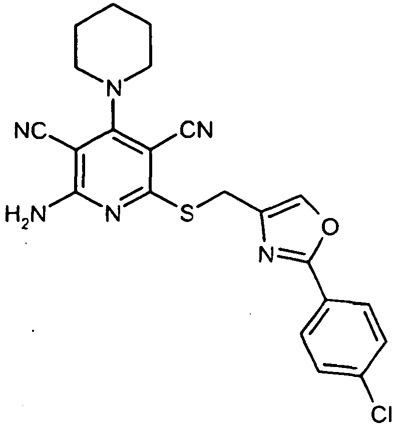

Die Titelverbindung wird analog zu Beispiel 1 aus den entsprechenden Edukten hergestellt.
Ausbeute: 26 mg (12% d. Th.)
¹H-NMR (400 MHz, DMSO-d₆): δ = 8.28 (s, 1H), 7.94 (d, 2H), 7.71-7.52 (br. s, 2H), 7.60 (d, 2H), 4.32 (s, 2H), 3.48 (br. s, 4H), 1.61 (br. s, 6H).
LC-MS (Methode 9): Rₜ = 1.47 min; MS (ESIpos): m/z = 451 [M+H]⁺.

### Beispiel 38

### 2-Amino-6-{[(1-oxido-6-piperidin-1-ylpyridin-3-yl)methyl]sulfanyl}-4-(piperidin-1-yl)-pyridin-3,5-dicarbonitril

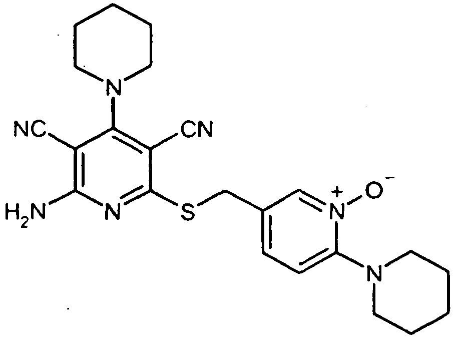

80 mg (0.22 mmol) der Verbindung aus Beispiel 12A werden in 1.9 ml Aceton suspendiert und mit 1. ml (10.99 mmol) Piperidin versetzt. Das Reaktionsgemisch wird 12 h bei RT gerührt. Nach Entfernen des Lösungsmittels am Rotationsverdampfer wird der Rückstand mittels präparativer HPLC gereinigt (Säule: YMC GEL ODS-AQ S-5 / 15 µm; Laufmittelgradient: Acetonitril/Wasser 10:90 → 95:5).
Ausbeute: 27 mg (26% d. Th.)
¹H-NMR (400 MHz, DMSO-d₆): δ = 8.33 (d, 1H), 7.96-7.61 (br. s, 2H), 7.35 (dd, 1H), 6.94 (d, 1H), 4.29 (s, 2H), 3.51-3.43 (br. s, 4H), 3.23-3.14 (br. s, 4H), 1.67-1.51 (br. s, 12H).
LC-MS (Methode 5): Rₜ = 3.41 min; MS (ESIpos): m/z = 450 [M+H]⁺.

### Beispiel 39

### 2-Amino-6-{[(1-oxido-6-pyrrolidin-1-ylpyridin-3-yl)methyl]sulfanyl}-4-(pyrrolidin-1-yl)-pyridin-3,5-dicarbonitril

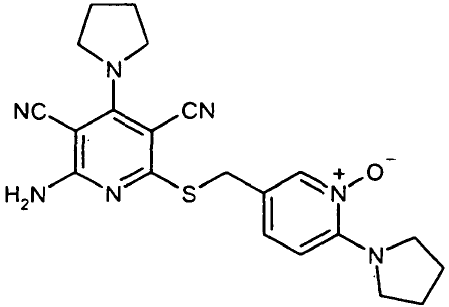

Die Titelverbindung wird analog zu Beispiel 38 ausgehend von Beispiel 12A und Pyrrolidin hergestellt.
Ausbeute: 22 mg (23% d. Th.)
¹H-NMR (400 MHz, DMSO-d₆): δ = 8.21 (s, 1H). 7.70-7.32 (br. s, 2H), 7.27 (dd, 1H), 6.77 (d, 1 H), 4.24 (s, 2H), 3.89-3.78 (br. s, 4H), 3.54-3.43 (br. s, 4H), 1.93-1.83 (br. s, 4H), 1.83-1.76 (br. s, 4H).
LC-MS (Methode 5): Rₜ = 2.67 min; MS (ESIpos): m/z = 422 [M+H]⁺.

### B. Bewertung der pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:
Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den Al-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-*N-*Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol.' 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 1**

| **Beispiel Nr.** | **EC₅₀ A1 [nM] (1 µM Forskolin)** | **EC₅₀ A2a [DM]** | **EC₅₀ A2b [nM]** |
|---|---|---|---|
| **1** | 1.3 | 686 | 116 |
| **2** | 0.5 | 385 | 44 |
| **4** | 12 | >3000 | >3000 |
| **6** | 45 | 1810 | >3000 |
| **27** | 0.3 | 63 | 0.9 |
| **30** | 5.7 | 1030 | 56 |
| **34** | 46 | >3000 | >3000 |
| **35** | 8.6 | >3000 | >3000 |
| **37** | 2.2 | 357 | 403 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Bestimmung der Löslichkeit

### Benötigte Reagenzien:

- PBS-Puffer pH 7.4: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N NaOH (z.B. Fa. Bernd Kraft GmbH, Art.-Nr. 01030.4000) in einen 1 Liter-Messkolben einwiegen, mit Wasser auffüllen und ca. 1 Stunde rühren;
- Acetatpuffer pH 4.6: 5.4 g Natriumacetat x 3 H₂O p.a. (z.B. Fa. Merck, Art.-Nr. 1.06267.0500) in einen 100 ml-Messkolben einwiegen, in 50 ml Wasser lösen, mit 2.4 g Eisessig versetzen, auf 100 ml mit Wasser auffüllen, pH-Wert überprüfen und falls notwendig auf pH 4.6 einstellen;
- Dimethylsulfoxid (z.B. Fa. Baker, Art.-Nr. 7157.2500);
- destilliertes Wasser.

### Herstellung der Kalibrierlösungen:

*Herstellung der Ausgangslösung für Kalibrierlösungen (Stammlösung):* In ein 2 ml Eppendorf-Safe-Lock Tube (Fa. Eppendorf, Art.-Nr. 0030 120.094) werden ca. 0.5 mg der Testsubstanz genau eingewogen, zu einer Konzentration von 600 µg/ml mit DMSO versetzt (z.B. 0.5 mg Substanz + 833 µl DMSO) und bis zur vollständigen Lösung mittels eines Vortexers geschüttelt.

*Kalibrierlösung 1 (20 µg*/*ml):* 34.4 µl der Stammlösung werden mit 1000 µl DMSO versetzt und homogenisiert.

*Kalibrierlösung 2 (2.5 µg*/*ml):* 100 µl der Kalibrierlösung 1 werden mit 700 µl DMSO versetzt und homogenisiert.

### Herstellung der Probenlösungen:

*Probenlösung für Löslichkeit bis 10 g*/*l in PBS-Puffer pH 7.4:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit PBS-Puffer pH 7.4 versetzt (z.B. 5 mg Substanz + 500 µl PBS-Puffer pH 7.4).

*Probenlösung für Löslichkeit bis 10 g*/*l in Acetatpuffer pH 4.6:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit Acetatpuffer pH 4.6 versetzt (z.B. 5 mg Substanz + 500 µl Acetatpuffer pH 4.6).

*Probenlösung für Löslichkeit bis 10 g*/*l in Wasser:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit Wasser versetzt (z.B. 5 mg Substanz + 500 µl Wasser).

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und 1:5, 1:100 und 1:1000 mit dem jeweils verwendeten Lösungsmittel (Wasser, PBS-Puffer 7.4 oder Acetatpuffer pH 4.6) verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/l ausgedrückt. Analysensequenz: 1) Kalibrierlösung 2.5 mg/ml; 2) Kalibrierlösung 20 µg/ml; 3) Probenlösung 1:5; 4) Probenlösung 1:100; 5) Probenlösung 1:1000.

### HPLC-Methode für Säüren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 50 mm x 2 mm, 5 µ; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 p; Temperatur: 30°C; Eluent A: Wasser + 5 ml Perchlorsäure/l; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### B-6. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparinisiert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC, Cₘₐₓ, T_{½} (Halbwertszeit) und CL (Clearance) mittels eines validierten pharmakokinetischen Rechenprogramms berechnet.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 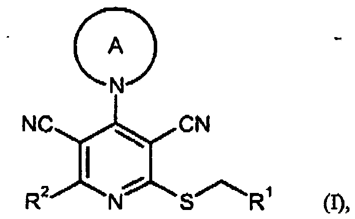 in welcher
der Ring A für einen gesättigten, 4- bis 7-gliedrigen, N-verknüpften Heterocyclus steht, der ein weiteres Ring-Heteroatom aus der Reihe N, O und S enthalten kann und der
(*i*) bis zu fünffach, gleich oder verschieden, mit (C₁-C₄)-Alkyl, das seinerseits ein- oder zweifach, gleich oder verschieden, mit Oxo, Hydroxy, (C₁-C₄)-Alkoxy, Amino, Mono-(C₁...C₄)-alkylamino, Di-(C₁-C₄)-alkylamino und/oder (C₃-C₆)-Cycloalkyl substituiert sein kann,
und/oder
(*ii*) ein- oder zweifach, gleich oder verschieden, mit Oxo, Thioxo, Hydroxy, (C₁-C₄)-Alkoxy, Amino, Mono-(C₁-C₄)-alkylamino, Di-(C₁-C₄)-alkylamino und/oder (C₃-C₆)-Cycloalkyl
substituiert sein kann,
R¹ für (C₆-C₁₀)-Aryl oder 5- bis 10-gliedriges Heteroaryl mit bis zu drei Ring-Heteroatomen aus der Reihe N, O und/oder S steht, welche jeweils
(*i*) ein- oder zweifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Amino, Meno-(C₁-C₆)-alkylamino, Di-(C₁-C₆)-alkylamino, Mono-(C₂-C₆)-alkenylamino, Carboxyl, (C₁-C₆)-Alkoxycarbonyl, Carbamoyl, Mono-(C₁-C₆)-alkylaminocarbonyl und Di-(C₁-C₆)-alkylaminocarbonyl
und/oder
(*ii*) mit Pyrrolidino, Piperidino, Morpholine, Piperazino, N'-(C₁-C₄)-Alkyl-piperazino oder einer Gruppe der Formel -L-R³, worin
L eine Bindung, NH oder O bedeutet
und
R³ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, (C₁-C₆)-Alkoxycarbonyl und Carboxyl substituiert sein können,
substituiert sein können, und
R² für Wasserstoff oder für (C₁-C₆)-Alkyl oder (C₁-C₆)-Alkoxy, welche jeweils mit Hydroxy, (C₁-C₄)-Alkoxy, Carboxyl, (C₁-C₄)-Alkoxycarbonyl oder bis zu dreifach mit Fluor substituiert sein können, steht
oder
R² für eine Gruppe der Formel -NR⁴R⁵ steht, worin
R⁴ und R⁵ gleich oder verschieden sind und unabhängig voneinander für Wasserstoff oder (C₁-C₆)-Alkyl, das ein- oder zweifach, gleich oder verschieden, mit Hydroxy, (C₁-C₄)-Alkoxy, Amino, Mono-(C₁-C₄)-alkylamino, Di-(C₁-C₄)-alkylamino, Carboxyl, (C₁-C₄)-Alkoxycarbonyl und/oder einem 4- bis 7-gliedrigen Heterocyclus substituiert sein kann, stehen,
wobei der genannte Heterocyclus ein oder zwei Ring-Heteroatome aus der Reihe N, O und/oder S enthält und seinerseits ein- oder zweifach, gleich oder verschieden, mit (C₁-C₄)-Alkyl, Hydroxy, Oxo und/oder (C₁-C₄)-Alkoxy substituiert sein kann,
oder
R⁴ und R⁵ zusammen mit dem Stickstoffatom, an das sie gebunden sind, einen 4-bis 7-gliedrigen Heterocyclus bilden, der ein weiteres Ring-Heteroatom aus der Reihe N, O und S enthalten und ein- oder zweifach, gleich oder verschieden, mit (C₁-C₄)-Alkyl, Hydroxy, Oxo, (C₁-C₄)-Alkoxy, Azetidino, Pyrrolidino, Piperidino und/oder Morpholino substituiert sein kann,
sowie ihre N-Oxide, Salze, Solvate, Salze der N-Oxide und Solvate der N-Oxide und Salze.

2. Verbindung der Formel (I) nach Anspruch 1, in welcher
R¹ für Phenyl oder 5- oder 6-gliedriges Heteroaryl mit bis zu drei Ring-Heteroatomen aus der Reihe N, O und/oder S steht, welche jeweils
(i) ein- oder zweifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Mono-(C₂-C₆)-alkenylamino, Carboxyl, (C₁-C₆)-Alkoxycarbonyl, Carbamoyl, Mono-(C₁-C₆)-alkylaminocarbonyl und Di-(C₁-C₆)-alkylaminocarbonyl
und/oder
(ii) mit Pyrrolidino, Piperidino, Morpholino, Piperazino, N'-(C₁-C₄)-Alkyl-piperazino oder einer Gruppe der Formel -L-R³, worin
L eine Bindung, NH oder O bedeutet
und
R³ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit einem Rest ausgewählt aus der Reihe Halogen, Nitro, Cyano, (C₁-C₆)-Alkyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, (C₁-C₆)-Alkoxycarbonyl und Carboxyl substituiert sein können,
substituiert sind,
oder
R¹ für N-Oxidopyridyl steht,
sowie ihre Salze, Solvate und Solvate der Salze.

3. Verbindung der Formel (I) nach Anspruch 1 oder 2, in welcher
der Ring A für einen gesättigten, 5- bis 7-gliedrigen, N-verknüpften Heterocyclus steht, der ein weiteres Ring-Heteroatom aus der Reihe N und O enthalten kann und der
(*i*) bis zu fünffach, gleich oder verschieden, mit (C₁-C₃)-Alkyl, das seinerseits ein- oder zweifach, gleich oder verschieden, mit Oxo, Hydroxy, (C₁-C₃)-Alkoxy, Amino, Mono-(C₁-C₃)-alkylamino, Di-(C₁-C₃)-alkylamino und/oder (C₃-C₅)-Cycloalkyl substituiert sein kann,
und/oder
(ii) ein- oder zweifach, gleich oder verschieden, mit Oxo, Hydroxy, (C₁-C₃)-Alkoxy, Amino, Mono-(C₁-C₃)-alkylamino, Di-(C₁-C₃)-alkylamino und/oder (C₃-C₅)-Cycloalkyl
substituiert sein kann,
R¹ für Phenyl oder 5- oder 6-gliedriges Heteroaryl mit bis zu drei Ring-Heteroatomen aus der Reihe N, O und/oder S steht, welche jeweils
(i) ein- oder zweifach, gleich oder verschieden, mit Fluor, Chlor, Cyano, (C₁-C₄)-Alkyl, Amino, Mono-(C₁-C₄)-alkylamino und/oder Di-(C₁-C₄)-alkylamino
und/oder
(ii) mit Morpholino, *N*'-(C₁-C₄)-Alkylpiperazino oder einer Gruppe der Formel -L-R³, worin
L eine Bindung oder NH bedeutet
und
R³ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit Fluor, Chlor, Cyano, (C₁-C₄)-Alkyl, Trifluormethyl, (C₁-C₄)-Alkoxy, Trifluormethoxy und/oder Carboxyl substituiert sein können,
substituiert sind,
oder
R¹ für N-Oxidopyridyl steht,
und
R² für Wasserstoff oder für (C₁-C₄)-Alkoxy, das bis zu dreifach mit Fluor substituiert sein kann, steht
oder
R² für eine Gruppe der Formel -NR⁴R⁵ steht, worin
R⁴ für Wasserstoff oder (C₁-C₄)-Alkyl, das mit Hydroxy, (C₁-C₄)-Alkoxy, Amino, Mono-(C₁-C₄)-alkylamino, Di-(C₁-C₄)-alkylamino, Carboxyl, (C₁-C₄)-Alkoxycarbonyl oder einem 5- oder 6-gliedrigen Heterocyclus substituiert sein kann, steht,
wobei der genannte Heterocyclus ein oder zwei Ring-Heteroatome aus der Reihe N und/oder O enthält und seinerseits ein- oder zweifach, gleich oder verschieden, mit Methyl, Ethyl, Hydroxy, Methoxy und/oder Ethoxy substituiert sein kann,
R⁵ für Wasserstoff oder Methyl steht
oder
R⁴ und R⁵ zusammen mit dem Stickstoffatom, an das sie gebunden sind, einen 5-oder 6-gliedrigen Heterocyclus bilden, der ein weiteres Ring-Heteroatom aus der Reihe N und O enthalten und ein- oder zweifach, gleich oder verschieden, mit Methyl, Ethyl, Hydroxy, Methoxy und/oder Ethoxy substituiert sein kann,
sowie ihre Salze, Solvate und Solvate der Salze.

4. Verbindung der Formel (I) nach Anspruch 1, 2 oder 3, in welcher
der Ring A für eine Gruppe der Formel 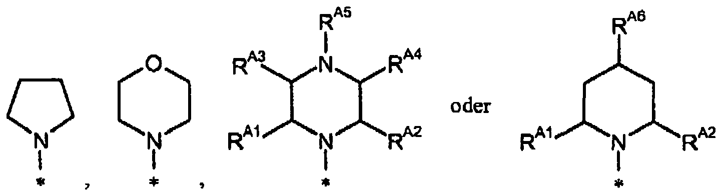 steht, worin
* die Verknüpfungsstelle mit dem Pyridin-Ring,
R^{A1}, R^{A2}, R^{A3} und R^{A4} unabhängig voneinander Wasserstoff oder Methyl,
R^{A5} Wasserstoff, Methyl, Ethyl, 2-Hydroxyethyl oder Cyclopropylmethyl
und
R^{A6} Wasserstoff, Methyl, Ethyl, Hydroxymethyl, 2-Hydroxyethyl, Cyclopropylmethyl, Hydroxy, Methoxy oder Ethoxy
bedeuten,
R¹ fUr Phenyl, Oxazolyl, Thiazolyl oder Pyridyl steht, welche jeweils
(*i*) ein- oder zweifach, gleich oder verschieden, mit Fluor, Chlor, Methyl und/oder Amino
oder
(ii) mit einer Gruppe der Formel -L-R³, worin
L eine Bindung oder NH bedeutet
und
R³ Phenyl oder Pyridyl bedeutet, welche jeweils ein- oder zweifach, gleich oder verschieden, mit Fluor, Chlor, Cyano, Methyl und/oder Methoxy substituiert sein können,
substituiert sind,
oder
R¹ für *N*-Oxidopyridyl steht,
und
R² für Wasserstoff, Methoxy oder eine Gruppe der Formel -NR⁴R⁵ steht, worin
R⁴ für Wasserstoff oder (C₁-C₄)-Alkyl, das mit Hydroxy, Amino, Methylamino, Ethylamino, Dimethylamino oder Diethylamino substituiert sein kann, steht,
R⁵ für Wasserstoff steht
oder
R⁴ und R⁵ zusammen mit dem Stickstoffatom, an das sie gebunden sind, eine Gruppe der Formel 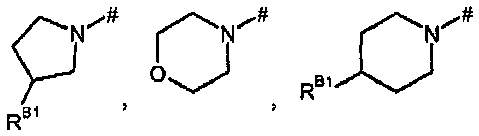 oder 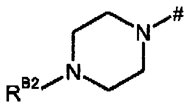 bilden, worin
# die Verknüpfungsstelle mit dem Pyridin-Ring,
R^{B1} Wasserstoff oder Hydroxy
und
R^{B2} Wasserstoff oder Methyl
bedeuten,
sowie ihre Salze, Solvate und Solvate der Salze.

5. Verfahren zur Herstellung von Verbindungen der Formel (I), wie in den Ansprüchen 1 bis 4 definiert, in welcher R² für NH₂ steht, **dadurch gekennzeichnet, dass** man die Verbindung der Formel (II) 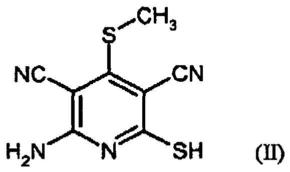 zunächst in einem inerten Lösungsmittel in Gegenwart einer Base mit einer Verbindung der Formel (III) 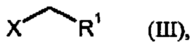 in welcher R¹ die in den Ansprüchen 1 bis 4 angegebene Bedeutung hat und
X für eine geeignete Abgangsgruppe wie Halogen, Mesylat, Tosylat oder Triflat steht,
zu einer Verbindung der Formel (IV) 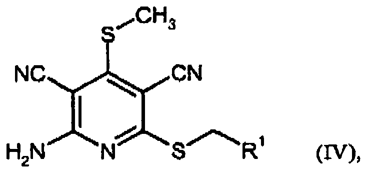 in welcher R¹ die oben angegebene Bedeutung hat,
reagiert und diese dann in einem inerten Lösungsmittel oder ohne weiteres Lösungsmittel mit einer Verbindung der Formel (V) 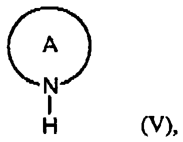 in welcher der Ring A die in den Ansprüchen 1 bis 4 angegebene Bedeutung hat,
zu einer Verbindung der Formel (I-A) 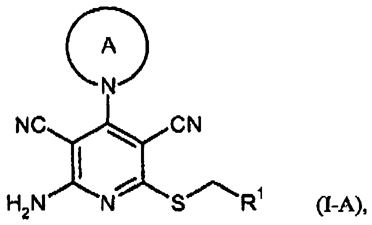 in welcher R¹ und der Ring A die zuvor angegebenen Bedeutungen haben,
umsetzt
und die Verbindungen der Formel (I-A) gegebenenfalls mit den entsprechenden (i) Lösungsmitteln und/oder (ii) Basen oder Säuren in ihre Solvate, Salze und/oder Solvate der Salze überführt.

6. Verbindung der Formel (I), wie in einem der Ansprüche 1 bis 4 definiert, zur Behandlung und/oder Prophylaxe von Krankheiten.

7. Verwendung einer Verbindung der Formel (I), wie in einem der Ansprüche 1 bis 4 definiert, zur Herstellung eines Arzneimittels zur Behandlung und/oder Prophylaxe von Hypertonie, koronarer Herzerkrankung, akutem Koronarsyndrom, Angina pectoris, Herzinsuffizienz, Myokardinfarkt und Vorhofflimmern.

8. Verwendung einer Verbindung der Formel (I), wie in einem der Ansprüche 1 bis 4 definiert, zur Herstellung eines Arzneimittels zur Behandlung und/oder Prophylaxe von Diabetes, Metabolischem Syndrom und Dyslipidämien.

9. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

10. Arzneimittel enthaltend eine Verbindung der Formel (I), wie in einem der Ansprüche 1 bis 4 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

11. Arzneimittel nach Anspruch 9 oder 10 zur Behandlung und/oder Prophylaxe von Hypertonie, koronarer Herzerkrankung, akutem Koronarsyndrom, Angina pectoris, Herzinsuffizienz, Myokardinfarkt und Vorhofflimmern.

12. Arzneimittel nach Anspruch 9 oder 10 zur Behandlung und/oder Prophylaxe von Diabetes, Metabolischem Syndrom und Dyslipidämien.

## Claims

1. Compound of the formula (I) 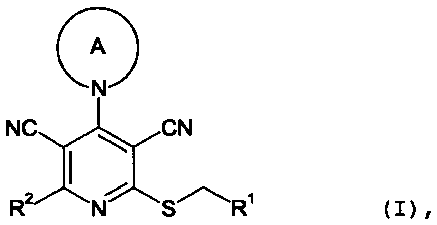 in which
ring A represents a 4- to 7-membered saturated heterocycle which is attached via nitrogen and which may contain a further ring heteroatom from the group consisting of N, O and S and which may be
(i) substituted up to five times by identical or different substituents from the group consisting of (C₁-C₄)-alkyl which for its part may be mono- or disubstituted by identical or different substituents from the group consisting of oxo, hydroxyl, (C₁-C₄)-alkoxy, amino, mono-(C₁-C₄)-alkylamino, di-(C₁-C₄)-alkylamino and (C₃-C₆)-cycloalkyl,
and/or
(ii) mono- or disubstituted by identical or different substituents from the group consisting of oxo, thioxo, hydroxyl, (C₁-C₄)-alkoxy, amino, mono-(C₁-C₄)-alkylamino, di-(C₁-C₄)-alkylamino and (C₃-C₆)-cycloalkyl,
R¹ represents (C₆-C₁₀)-aryl or 5- to 10-membered heteroaryl having up to three ring heteroatoms from the group consisting of N, O and S, each of which radicals may be
(i) mono- or disubstituted by identical or different radicals selected from the group consisting of halogen, nitro, cyano, (C₁-C₆)-alkyl, trifluoromethyl, hydroxyl, (C₁-C₆)-alkoxy, amino, mono-(C₁-C₆)-alkylamino, di-(C₁-C₆)-alkylamino, mono-(C₁-C₆)-alkenylamino, carboxyl, (C₁-C₆)-alkoxycarbonyl, carbamoyl, mono-(C₁-C₆)-alkylaminocarbonyl and di-(C₁-C₆)-alkylaminocarbonyl
and/or
(ii) substituted by pyrrolidino, piperidino, morpholino, piperazino, N'-(C₁-C₄)-alkylpiperazino or a group of the formula -L-R³ in which
L represents a bond, NH or O
and
R³ represents phenyl or 5- or 6-membered heteroaryl having up to three ring heteroatoms from the group consisting of N, O and S, each of which radicals may be mono- to trisubstituted by identical or different radicals selected from the group consisting of halogen, nitro, cyano, (C₁-C₆)-alkyl, trifluoromethyl, hydroxyl, (C₁-C₆)-alkoxy, difluoromethoxy, trifluoromethoxy, amino, mono-(C₁-C₆)-alkylamino, di-(C₁-C₆)-alkylamino, (C₁-C₆)-alkoxycarbonyl and carboxyl,
and
R² represents hydrogen or represents (C₁-C₆)-alkyl or (C₁-C₆)-alkoxy, each of which radicals may be substituted by hydroxyl, (C₁-C₄)-alkoxy, carboxyl, (C₁-C₄)-alkoxycarbonyl or up to three times by fluorine
or
R² represents a group of the formula -NR⁴R⁵ in which
R⁴ and R⁵ are identical or different and independently of one another represent hydrogen or (C₁-C₆)-alkyl which may be mono- or disubstituted by identical or different radicals from the group consisting of hydroxyl, (C₁-C₄)-alkoxy, amino, mono-(C₁-C₄)-alkylamino, di-(C₁-C₄)-alkylamino, carboxyl, (C₁-C₄)-alkoxycarbonyl and a 4- to 7-membered heterocycle,
where the heterocycle mentioned contains one or two ring heteroatoms from the group consisting of N, **O** and S and for its part may be mono- or disubstituted by identical or different radicals from the group consisting of (C₁-C₄)-alkyl, hydroxyl, oxo and (C₁-C₄)-alkoxy,
or
R⁴ and R⁵ together with the nitrogen atom, to which they are attached, form a 4- to 7-membered heterocycle which may contain a further ring heteroatom from the group consisting of N, **O** or S and may be mono- or disubstituted by identical or different radicals from the group consisting of (C₁-C₄)-alkyl, hydroxyl, oxo, (C₁-C₄)-alkoxy, azetidino, pyrrolidino, piperidino and morpholino,
and N-oxides, salts, solvates, salts of the N-oxides and solvates of the N-oxides and salts thereof.

2. Compound of the formula (I) according to Claim 1 in which
R¹ represents phenyl or 5- or 6-membered heteroaryl having up to three ring heteroatoms from the group consisting of N, O and S, each of which radicals is
(i) mono- or disubstituted by identical or different radicals selected from the group consisting of halogen, nitro, cyano, (C₁-C₆)-alkyl, trifluoromethyl, hydroxyl, (C₁-C₆)-alkoxy, amino, mono-(C₁-C₆)-alkylamino, di-(C₁-C₆)-alkylamino, mono-(C₂-C₆)-alkenylamino, carboxyl, (C₁-C₆)-alkoxycarbonyl, carbamoyl, mono-(C₁-C₆)-alkylaminocarbonyl and di-(C₁-C₆)-alkylaminocarbonyl
and/or
(ii) substituted by pyrrolidino, piperidino, morpholino, piperazino, N'-(C₁-C₄)-alkylpiperazino or a group of the formula -L-R³ in which
L represents a bond, NH or O
and
R³ represents phenyl or 5- or 6-membered heteroaryl having up to three ring heteroatoms from the group consisting of N, **O** and S, each of which radicals may be mono- to trisubstituted by identical or different radicals selected from the group consisting of halogen, nitro, cyano, (C₁-C₆)-alkyl, trifluoromethyl, hydroxyl, (C₁-C₆)-alkoxy, difluoromethoxy, trifluoromethoxy, amino, mono-(C₁-C₆)-alkylamino, di-(C₁-C₆)-alkylamino, (C₁-C₆)-alkoxycarbonyl and carboxyl,
or
R¹ represents N-oxidopyridyl,
and salts, solvates and solvates of the salts thereof.

3. Compound of the formula (I) according to Claim 1 or 2 in which
ring A represents a 5- to 7-membered saturated heterocycle which is attached via nitrogen and which may contain a further ring heteroatom from the group consisting of N and O and which may be
(i) substituted up to five times by identical or different substituents from the group consisting of (C₁-C₃)-alkyl which for its part may be mono- or disubstituted by identical or different substituents from the group consisting of oxo, hydroxyl, (C₁-C₃)-alkoxy, amino, mono-(C₁-C₃)-alkylamino, di-(C₁-C₃)-alkylamino and (C₃-C₅)-cycloalkyl,
and/or
(ii) mono- or disubstituted by identical or different radicals selected from the group consisting of oxo, hydroxyl, (C₁-C₃)-alkoxy, amino, mono-(C₁-C₃)-alkylamino, di-(C₁-C₃)-alkylamino and (C₃-C₅)-cycloalkyl,
R¹ represents phenyl or 5- or 6-membered heteroaryl having up to three ring heteroatoms from the group consisting of N, O and S, each of which radicals is
(*i*) mono- or disubstituted by identical or different radicals selected from the group consisting of fluorine, chlorine, cyano, (C₁-C₄)-alkyl, amino, mono-(C₁-C₄)-alkylamino and di-(C₁-C₄)-alkylamino
and/or
(*ii*) substituted by morpholino, *N'*-(C₁-C₄)-alkylpiperazino or a group of the formula -L-R³ in which
L represents a bond or NH
and
R³ represents phenyl or 5- or 6-membered heteroaryl having up to three ring heteroatoms from the group consisting of N, O and S, each of which radicals may be mono- to trisubstituted by identical or different radicals selected from the group consisting of fluorine, chlorine, cyano, (C₁-C₄)-alkyl, trifluoromethyl, (C₁-C₄)-alkoxy, trifluoromethoxy and carboxyl,
or
R¹ represents N-oxidopyridyl,
and
R² represents hydrogen or represents (C₁-C₄)-alkoxy which may be substituted up to three times by fluorine
or
R² represents a group of the formula -NR⁴R⁵ in which
R⁴ represents hydrogen or (C₁-C₄)-alkyl which may be substituted by hydroxyl, (C₁-C₄)-alkoxy, amino, mono-(C₁-C₄)-alkylamino, di-(C₁-C₄)-alkylamino, carboxyl, (C₁-C₄)-alkoxycarbonyl or a 5-or 6-membered heterocycle,
where the heterocycle mentioned contains one or two ring heteroatoms from the group consisting of N and O and for its part may be mono- or disubstituted by identical or different radicals from the group consisting of methyl, ethyl, hydroxyl, methoxy and ethoxy,
R⁵ represents hydrogen or methyl
or
R⁴ and R⁵ together with the nitrogen atom, to which they are attached, form a 5- or 6-membered heterocycle which may contain a further ring heteroatom from the group consisting of N or **O** and may be mono- or disubstituted by identical or different radicals from the group consisting of methyl, ethyl, hydroxyl, methoxy and ethoxy,
and salts, solvates and solvates of the salts thereof.

4. Compound of the formula (I) according to Claim 1, 2 or 3 in which
ring A represents a group of the formula 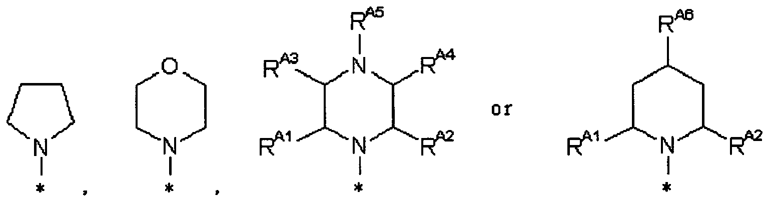 in which
* denotes the point of attachment to the pyridine ring,
R^{A1}, R^{A2}, R^{A3} and R^{A4} independently of one another represent hydrogen or methyl,
R^{A5} represents hydrogen, methyl, ethyl, 2-hydroxyethyl or cyclopropylmethyl
and
R^{A6} represents hydrogen, methyl, ethyl, hydroxymethyl, 2-hydroxyethyl, cyclopropylmethyl, hydroxyl, methoxy or ethoxy,
R¹ represents phenyl, oxazolyl, thiazolyl or pyridyl, each of which radicals is
(i) mono- or disubstituted by identical or different radicals selected from the group consisting of fluorine, chlorine, methyl and amino
or
(ii) substituted by a group of the formula - L-R³ in which
L represents a bond or NH
and
R³ represents phenyl or pyridyl, each of which radicals may be mono- or disubstituted by identical or different radicals selected from the group consisting of fluorine, chlorine, cyano, methyl and methoxy,
or
R¹ represents N-oxidopyridyl,
and
R² represents hydrogen, methoxy or a group of the formula -NR⁴R⁵ in which
R⁴ represents hydrogen or (C₁-C₄)-alkyl which may be substituted by hydroxyl, amino, methylamino, ethylamino, dimethylamino or diethylamino,
R⁵ represents hydrogen
or
R⁴ and R⁵ together with the nitrogen atom to which they are attached form a group of the formula 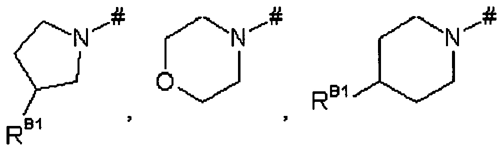 or 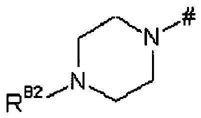 in which
# denotes the point of attachment to the pyridine ring,
R^{B1} represents hydrogen or hydroxyl
and
R^{B2} represents hydrogen or methyl,
and salts, solvates and solvates of the salts thereof.

5. Process for preparing the compounds of the formula (I) as defined in any of Claims 1 to 4 in which R² represents NH₂, **characterized in that** the compound of the formula (II) 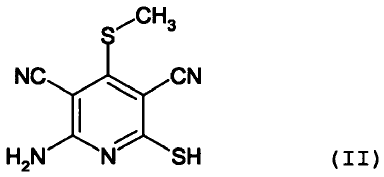 is initially, in an inert solvent in the presence of a base, reacted with a compound of the formula (III) 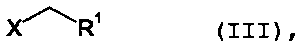 in which R¹ has the meaning given in Claims 1 to 4 and
X represents a suitable leaving group such as halogen, mesylate, tosylate or triflate,
to give a compound of the formula (IV) 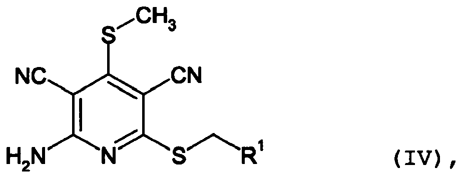 in which R¹ has the meaning given above,
and this compound is then, in an inert solvent or without further solvent, reacted with a compound of the formula (V) 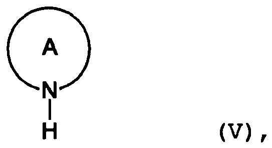 in which ring A has the meaning given in any of Claims 1 to 4,
to give a compound of the formula (I-A) 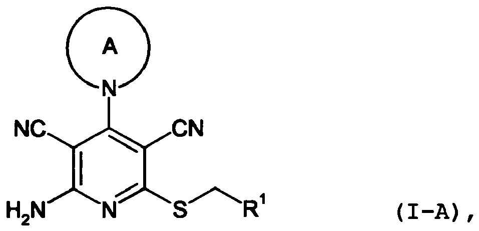 in which R¹ and ring A have the meanings given above,
and the compounds of the formula (I-A) are, if appropriate, converted with the appropriate (i) solvents and/or (ii) bases or acids into their solvates, salts and/or solvates of the salts.

6. Compound of the formula (I) as defined in any of Claims 1 to 4 for the treatment and/or prophylaxis of diseases.

7. Use of a compound of the formula (I) as defined in any of Claims 1 to 4 for preparing a medicament for the treatment and/or prophylaxis of hypertension, coronary heart disease, acute coronary syndrome, angina pectoris, heart failure, myocardial infarction and atrial fibrillation.

8. Use of a compound of the formula (I) as defined in any of Claims 1 to 4 for preparing a medicament for the treatment and/or prophylaxis of diabetes, metabolic syndrome and dyslipidemias.

9. Medicament comprising a compound of the formula (I) as defined in any of Claims 1 to 4 in combination with an inert nontoxic pharmaceutically suitable auxiliary.

10. Medicament comprising a compound of the formula (I) as defined in any of Claims 1 to 4 in combination with one or more further active compounds selected from the group consisting of lipid metabolism-modifying active compounds, antidiabetics, antihypertensive drugs and antithrombotic drugs.

11. Medicament according to Claim 9 or 10 for the treatment and/or prophylaxis of hypertension, coronary heart disease, acute coronary syndrome, angina pectoris, heart failure, myocardial infarction and atrial fibrillation.

12. Medicament according to Claim 9 or 10 for the treatment and/or prophylaxis of diabetes, metabolic syndrome and dyslipidemias.

## Revendications

1. Composé de formule (I) 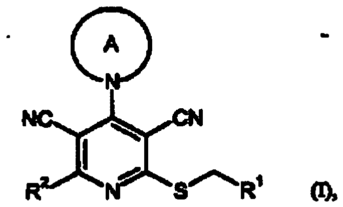 dans laquelle
le cycle A représente un hétérocycle saturé, de 4 à 7 éléments, relié à N, qui peut contenir un hétéroatome de cycle supplémentaire de la série N, O et S, et qui peut être substitué
(i) jusqu'à cinq fois, de manière identique ou différente, avec alkyle en (C₁-C₄), qui peut de son côté être substitué une ou deux fois, de manière identique ou différente, avec oxo, hydroxy, alcoxy en (C₁-C₄), amino, mono-alkylamino en (C₁-C₄), di-alkylamino en (C₁-C₄) et/ou cycloalkyle en (C₃-C₆),
et/ou
(ii) une ou deux fois, de manière identique ou différente, avec oxo, thioxo, hydroxy, alcoxy en (C₁-C₄), amino, mono-alkylamino en (C₁-C₄), di-alkylamino en (C₁-C₄) et/ou cycloalkyle en (C₃-C₆),
R¹ représente aryle en (C₆-C₁₀) ou hétéroaryle de 5 à 10 éléments contenant jusqu'à trois hétéroatomes de cycle de la série N, O et/ou S, qui peuvent chacun être substitués
(i) une ou deux fois, de manière identique ou différente, avec un radical choisi dans la série halogène, nitro, cyano, alkyle en (C₁-C₆), trifluorométhyle, hydroxy, alcoxy en (C₁-C₆), amino, mono-alkylamino en (C₁-C₆), di-alkylamino en (C₁-C₆), mono-alcénylamino en (C₂-C₆), carboxyle, alcoxycarbonyle en (C₁-C₆), carbamoyle, mono-alkylaminocarbonyle en (C₁-C₆) et di-alkylaminocarbonyle en (C₁-C₆),
et/ou
(ii) avec pyrrolidino, pipéridino, morpholino, pipérazino, N'-alkylpipérazino en (C₁-C₄) ou un groupe de formule -L-R³, dans laquelle
L signifie une liaison, NH ou O,
et
R³ signifie phényle ou hétéroaryle de 5 ou 6 éléments contenant jusqu'à trois hétéroatomes de cycle de la série N, O et/ou S, qui peuvent chacun être substitués une à trois fois, de manière identique ou différente, avec un radical choisi dans la série halogène, nitro, cyano, alkyle en (C₁-C₆), trifluorométhyle, hydroxy, alcoxy en (C₁-C₆), difluorométhoxy, trifluorométhoxy, amino, mono-alkylamino en (C₁-C₆), di-alkylamino en (C₁-C₆), alcoxycarbonyle en (C₁-C₆) et carboxyle,
et
R² représente hydrogène ou alkyle en (C₁-C₆) ou alcoxy en (C₁-C₆), qui peuvent chacun être substitués avec hydroxy, alcoxy en (C₁-C₄), carboxyle, alcoxycarbonyle en (C₁-C₄) ou jusqu'à trois fois avec fluor,
ou
R² représente un groupe de formule -NR⁴R⁵, dans laquelle
R⁴ et R⁵ sont identiques ou différents et représentent indépendamment l'un de l'autre hydrogène ou alkyle en (C₁-C₆), qui peut être substitué une ou deux fois, de manière identique ou différente, avec hydroxy, alcoxy en (C₁-C₄), amino, mono-alkylamino en (C₁-C₄), di-alkylamino en (C₁-C₄), carboxyle, alcoxycarbonyle en (C₁-C₄) et/ou un hétérocycle de 4 à 7 éléments,
l'hétérocycle mentionné contenant un ou deux hétéroatomes de cycle de la série N, O et/ou S et pouvant être substitué de son côté une ou deux fois, de manière identique ou différente, avec alkyle en (C₁-C₄), hydroxy, oxo et/ou alcoxy en (C₁-C₄),
ou
R⁴ et R⁵ forment ensemble avec l'atome d'azote auquel ils sont reliés un hétérocycle de 4 à 7 éléments, qui peut contenir un hétéroatome de cycle supplémentaire de la série N, O et S et être substitué une ou deux fois, de manière identique ou différente, avec alkyle en (C₁-C₄), hydroxy, oxo, alcoxy en (C₁-C₄), azétidino, pyrrolidino, pipéridino et/ou morpholino, ainsi que leurs N-oxydes, sels, solvates, sels des N-oxydes et solvates des N-oxydes et des sels.

2. Composé de formule (I) selon la revendication 1, dans lequel
R¹ représente phényle ou hétéroaryle de 5 ou 6 éléments contenant jusqu'à trois hétéroatomes de cycle de la série N, O et/ou S, qui peuvent chacun être substitués
(i) une ou deux fois, de manière identique ou différente, avec un radical choisi dans la série halogène, nitro, cyano, alkyle en (C₁-C₆), trifluorométhyle, hydroxy, alcoxy en (C₁-C₆), amino, mono-alkylamino en (C₁-C₆), di-alkylamino en (C₁-C₆), mono-alcénylamino en (C₂-C₆), carboxyle, alcoxycarbonyle en (C₁-C₆), carbamoyle, mono-alkylaminocarbonyle en (C₁-C₆) et di-alkylaminocarbonyle en (C₁-C₆),
et/ou
(ii) avec pyrrolidino, pipéridino, morpholino, pipérazino, N'-alkylpipérazino en (C₁-C₄) ou un groupe de formule -L-R³, dans laquelle
L signifie une liaison, NH ou O,
et
R³ signifie phényle ou hétéroaryle de 5 ou 6 éléments contenant jusqu'à trois hétéroatomes de cycle de la série N, O et/ou S, qui peuvent chacun être substitués une à trois fois, de manière identique ou différente, avec un radical choisi dans la série halogène, nitro, cyano, alkyle en (C₁-C₆), trifluorométhyle, hydroxy, alcoxy en (C₁-C₆), difluorométhoxy, trifluorométhoxy, amino, mono-alkylamino en (C₁-C₆), di-alkylamino en (C₁-C₆), alcoxycarbonyle en (C₁-C₆) et carboxyle,
ou
R¹ représente N-oxydopyridyle,
ainsi que leurs sels, solvates et solvates des sels.

3. Composé de formule (I) selon la revendication 1 ou 2, dans lequel
le cycle A représente un hétérocycle saturé, de 5 à 7 éléments, relié à N, qui peut contenir un hétéroatome de cycle supplémentaire de la série N et O, et qui peut être substitué
(i) jusqu'à cinq fois, de manière identique ou différente, avec alkyle en (C₁-C₃), qui peut de son côté être substitué une ou deux fois, de manière identique ou différente, avec oxo, hydroxy, alcoxy en (C₁-C₃), amino, mono-alkylamino en (C₁-C₃), di-alkylamino en (C₁-C₃) et/ou cycloalkyle en (C₃-C₅),
et/ou
(ii) une ou deux fois, de manière identique ou différente, avec oxo, hydroxy, alcoxy en (C₁-C₃), amino, mono-alkylamino en (C₁-C₃), di-alkylamino en (C₁-C₃) et/ou cycloalkyle en (C₃-C₅),
R¹ représente phényle ou hétéroaryle de 5 ou 6 éléments contenant jusqu'à trois hétéroatomes de cycle de la série N, O et/ou S, qui peuvent chacun être substitués
(i) une ou deux fois, de manière identique ou différente, avec fluor, chlore, cyano, alkyle en (C₁-C₄), amino, mono-alkylamino en (C₁-C₄) et/ou di-alkylamino en (C₁-C₄),
et/ou
(ii) avec morpholino, N'-alkyle en (C₁-C₄)-pipérazino ou un groupe de formule -L-R³, dans laquelle L signifie une liaison ou NH,
et
R³ signifie phényle ou hétéroaryle de 5 ou 6 éléments contenant jusqu'à trois hétéroatomes de cycle de la série N, O et/ou S, qui peuvent chacun être substitués une à trois fois, de manière identique ou différente, avec fluor, chlore, cyano, alkyle en (C₁-C₄), trifluorométhyle, alcoxy en (C₁-C₄), trifluorométhoxy et/ou carboxyle,
ou
R¹ représente N-oxydopyridyle,
et
R² représente hydrogène ou alcoxy en (C₁-C₄), qui peut être substitué jusqu'à trois fois avec fluor,
ou
R² représente un groupe de formule -NR⁴R⁵, dans laquelle
R⁴ représente hydrogène ou alkyle en (C₁-C₄), qui peut être substitué avec hydroxy, alcoxy en (C₁-C₄), amino, mono-alkylamino en (C₁-C₄), di-alkylamino en (C₁-C₄), carboxyle, alcoxycarbonyle en (C₁-C₄) ou un hétérocycle de 5 ou 6 éléments,
l'hétérocycle mentionné contenant un ou deux hétéroatomes de cycle de la série N et/ou O et pouvant être substitué de son côté une ou deux fois, de manière identique ou différente, avec méthyle, éthyle, hydroxy, méthoxy et/ou éthoxy,
R⁵ représente hydrogène ou méthyle,
ou
R⁴ et R⁵ forment ensemble avec l'atome d'azote auquel ils sont reliés un hétérocycle de 5 ou 6 éléments, qui peut contenir un hétéroatome de cycle supplémentaire de la série N et O et être substitué une ou deux fois, de manière identique ou différente, avec méthyle, éthyle hydroxy, méthoxy et/ou éthoxy,
ainsi que leurs sels, solvates et solvates des sels.

4. Composé de formule (I) selon la revendication 1, 2 ou 3, dans lequel
le cycle A représente un groupe de formule 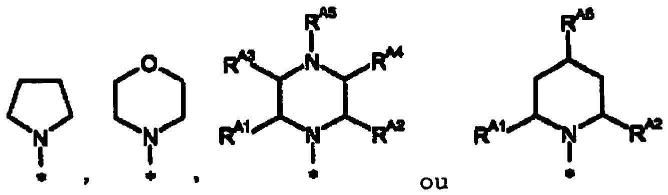 dans lesquelles
* signifie l'emplacement de liaison avec le cycle pyridine,
R^{A1}, R^{A2}, R^{A3} et R^{A4} signifient indépendamment les uns des autres hydrogène ou méthyle,
R^{A5} signifie hydrogène, méthyle, éthyle, 2-hydroxyéthyle ou cyclopropylméthyle
et
R^{A6} signifie hydrogène, méthyle, éthyle, hydroxyméthyle, 2-hydroxyéthyle, cyclopropylméthyle, hydroxy, méthoxy ou éthoxy,
R¹ représente phényle, oxazolyle, thiazolyle ou pyridyle, qui peuvent chacun être substitués
(i) une ou deux fois, de manière identique ou différente, avec fluor, chlore, méthyle et/ou amino,
ou
(ii) avec un groupe de formule -L-R³, dans laquelle L signifie une liaison ou NH,
et
R³ signifie phényle ou pyridyle, qui peuvent chacun être substitués une ou deux fois, de manière identique ou différente, avec fluor, chlore, cyano, méthyle et/ou méthoxy,
ou
R¹ représente N-oxydopyridyle,
et
R² représente hydrogène, méthoxy ou un groupe de formule -NR⁴R⁵, dans laquelle
R⁴ représente hydrogène ou alkyle en (C₁-C₄), qui peut être substitué avec hydroxy, amino, méthylamino, éthylamino, diméthylamino ou diéthylamino,
R⁵ représente hydrogène,
ou
R⁴ et R⁵ forment ensemble avec l'atome d'azote auquel ils sont reliés un groupe de formule 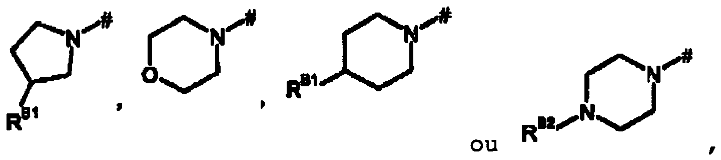 dans lesquelles
# signifie l'emplacement de liaison avec le cycle pyridine,
R^{B1} signifie hydrogène ou hydroxy,
et
R^{B2} signifie hydrogène ou méthyle,
ainsi que leurs sels, solvates et solvates des sels.

5. Procédé de fabrication de composés de formule (I), tels que définis dans les revendications 1 à 4, dans lesquels R² représente NH₂, **caractérisé en ce que** le composé de formule (II) 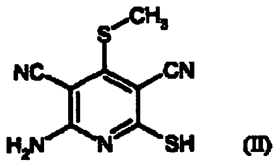 est tout d'abord mis en réaction dans un solvant inerte en présence d'une base avec un composé de formule (III) 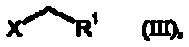 dans laquelle R¹ a la signification donnée dans les revendications 1 à 4, et
X représente un groupe partant approprié, tel qu'halogène, mésylate, tosylate ou triflate,
pour former un composé de formule (IV) 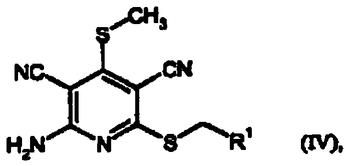 dans laquelle R¹ a la signification donnée précédemment, puis celui-ci est mis en réaction dans un solvant inerte ou sans solvant supplémentaire avec un composé de formule (V) 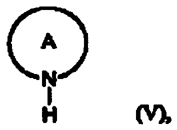 dans laquelle le cycle A a la signification donnée dans les revendications 1 à 4,
pour former un composé de formule (I-A) 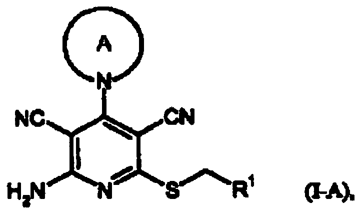 dans laquelle R¹ et le cycle A ont les significations données précédemment,
et les composés de formule (I-A) sont éventuellement transformés avec (i) les solvants et/ou (ii) les bases ou acides appropriés en leurs solvates, sels et/ou solvates des sels.

6. Composé de formule (I), tel que défini dans l'une quelconque des revendications 1 à 4, pour le traitement et/ou la prophylaxie de maladies.

7. Utilisation d'un composé de formule (I), tel que défini dans l'une quelconque des revendications 1 à 4, pour la fabrication d'un médicament pour le traitement et/ou la prophylaxie de l'hypertension, de la cardiopathie coronarienne, du syndrome coronarien aigu, de l'angine de poitrine, de l'insuffisance cardiaque, de l'infarctus du myocarde et de la fibrillation auriculaire.

8. Utilisation d'un composé de formule (I), tel que défini dans l'une quelconque des revendications 1 à 4, pour la fabrication d'un médicament pour le traitement et/ou la prophylaxie du diabète, du syndrome métabolique et des dyslipidémies.

9. Médicament contenant un composé de formule (I), tel que défini dans l'une quelconque des revendications 1 à 4, en combinaison avec un adjuvant inerte, non toxique, pharmaceutiquement approprié.

10. Médicament contenant un composé de formule (I), tel que défini dans l'une quelconque des revendications 1 à 4, en combinaison avec un ou plusieurs agents actifs supplémentaires choisis dans le groupe constitué par les agents actifs modifiant le métabolisme des lipides, les antidiabétiques, les agents actifs abaissant la tension artérielle et les agents actifs antithrombotiques.

11. Médicament selon la revendication 9 ou 10 pour le traitement et/ou la prophylaxie de l'hypertension, de la cardiopathie coronarienne, du syndrome coronarien aigu, de l'angine de poitrine, de l'insuffisance cardiaque, de l'infarctus du myocarde et de la fibrillation auriculaire.

12. Médicament selon la revendication 9 ou 10 pour le traitement et/ou la prophylaxie du diabète, du syndrome métabolique et des dyslipidémies.
